# EUROPEAN PATENT SPECIFICATION

(11) **EP 2 300 614 B1**
(45) Date of publication and mention of the grant of the patent: **28.10.2015**
(21) Application number: 09707233.4
(22) Date of filing: 03.02.2009
(51) Int. Cl.: C12N 15/62, C07K 14/435

(54) **METHODS AND COMPOSITIONS FOR TREATMENT OF MITOCHONDRIAL DISORDERS**
VERFAHREN UND ZUSAMMENSETZUNGEN ZUR BEHANDLUNG MITOCHONDRIALER STÖRUNGEN
PROCÉDÉS ET COMPOSITIONS DE TRAITEMENT DES MALADIES MITOCHONDRIALES

(30) Priority: 04.02.2008 US 25931 P
(43) Date of publication of application: 30.03.2011
(73) Proprietor: Yissum Research Development Company of the Hebrew University of Jerusalem Ltd., Jerusalem 91390 (IL); Hadasit Medical Research Services and Development Ltd., Jerusalem 91120 (IL)
(72) Inventor: GALSKI-LORBERBOUM, Haya, Jerusalem 97890 (IL); RAPOPORT, Matan, Maccabim 71908 (IL); ELPELEG, OrIy, Jerusalem 96263 (IL); SAADA, Ann, Jerusalem 93825 (IL)
(74) Representative: Oxley, Rachel Louise
(86) International application number: PCT/IL2009/000126
(87) International publication number: WO 2009/098682

(56) References cited:
- RAPOPORT M ET AL: "Enzyme replacement therapy for mitochondrial disorders - The lipoamide dehydrogenase deficiency as a model" JOURNAL OF INHERITED METABOLIC DISEASE, vol. 29, no. Suppl. 1, August 2006 (2006-08), page 15, XP009118346 & 10TH INTERNATIONAL CONGRESS OF INBORN ERRORS OF METABOLISM (ICIEM); CHIBA, JAPAN; SEPTEMBER 12 -16, 2006 ISSN: 0141-8955
- ANGDISEN J ET AL: "Mitochondrial trifunctional protein defects: molecular basis and novel therapeutic approaches." CURRENT DRUG TARGETS. IMMUNE, ENDOCRINE AND METABOLIC DISORDERS MAR 2005, vol. 5, no. 1, March 2005 (2005-03), pages 27-40, XP009118345 ISSN: 1568-0088
- DEL GAIZO V ET AL: "A novel TAT-mitochondrial signal sequence fusion protein is processed, stays in mitochondria, and crosses the placenta" MOLECULAR THERAPY, ACADEMIC PRESS, SAN DIEGO, CA, US, vol. 7, 1 June 2003 (2003-06-01), pages 720-730, XP002986584 ISSN: 1525-0016
- DEL GAIZO V ET AL: "Targeting proteins to mitochondria using TAT" MOLECULAR GENETICS AND METABOLISM, ACADEMIC PRESS, SAN DIEGO, CA, US, vol. 80, no. 1-2, 1 September 2003 (2003-09-01), pages 170-180, XP002986585 ISSN: 1096-7192
- TORO ANA ET AL: "TAT-mediated intracellular delivery of purine nucleoside phosphorylase corrects its deficiency in mice." THE JOURNAL OF CLINICAL INVESTIGATION OCT 2006, vol. 116, no. 10, October 2006 (2006-10), pages 2717-2726, XP002532521 ISSN: 0021-9738
- SHAHARYAR M KHAN ET AL: "Development of Mitochondrial Gene Replacement Therapy" JOURNAL OF BIOENERGETICS AND BIOMEMBRANES, KLUWER ACADEMIC PUBLISHERS-PLENUM PUBLISHERS, NE, vol. 36, no. 4, 1 August 2004 (2004-08-01), pages 387-393, XP019280561 ISSN: 1573-6881
- RAPOPORT MATAN ET AL: "TAT-mediated delivery of LAD restores pyruvate dehydrogenase complex activity in the mitochondria of patients with LAD deficiency." MOLECULAR THERAPY : THE JOURNAL OF THE AMERICAN SOCIETY OF GENE THERAPY APR 2008, vol. 16, no. 4, April 2008 (2008-04), pages 691-697, XP002532522 ISSN: 1525-0024
- BACKMAN J T ET AL: "Lack of correlation between in vitro and in vivo studies on the effects of tangeretin and tangerine juice on midazolam hydroxylation.", CLINICAL PHARMACOLOGY AND THERAPEUTICS APR 2000, vol. 67, no. 4, April 2000 (2000-04), pages 382-390, ISSN: 0009-9236
- P. M. Vyas ET AL: "A TAT-Frataxin fusion protein increases lifespan and cardiac function in a conditional Friedreich's ataxia mouse model", Human Molecular Genetics, vol. 21, no. 6, 23 November 2011 (2011-11-23), pages 1230-1247, XP055166451, ISSN: 0964-6906, DOI: 10.1093/hmg/ddr554

## Description

### FIELD OF THE INVENTION

The present invention concerns in general fusion proteins comprising a membrane-transferring moiety and an enzymatic moiety for use in methods of treating disease.

### BACKGROUND OF THE INVENTION

### Mitochondrial metabolic disorders

Mitochondria play a major and critical role in cellular homeostasis. They participate in intracellular signaling, apoptosis and perform numerous biochemical tasks, such as pyruvate oxidation, the Krebs cycle, and metabolism of amino acids, fatty acids, nucleotides and steroids. One crucial task is their role in cellular energy metabolism.

This includes β-oxidation of fatty acids and production of ATP by means of the electron-transport chain and the oxidative-phosphorylation system (Chinnery 2003).

The mitochondrial respiratory chain consists of five multi-subunit protein complexes embedded in the inner membrane, comprising: complex I (NADH-ubiquinone oxidoreductase), complex II (succinate-ubiquinone oxidoreductase), complex III (ubiquinol-ferricytochrome c oxidoreductase), complex IV (cytochrome c oxidoreductase), and complex V (F1F0 ATPase).

Most of the approximately 900 gene products in the mitochondria are encoded by nuclear DNA (nDNA); mtDNA contains only 13 protein encoding genes. Most of these polypeptides are synthesized with a mitochondrial targeting sequence (MTS), which allows their import from the cytoplasm into mitochondria through the translocation machinery (TOM/TIM). Once entering the mitochondria, the MTS is recognized and cleaved off, allowing for proper processing and, if necessary, assembly into mitochondrial enzymatic complexes (Chinnery, 2003).

### LAD deficiency

One of these imported proteins is Lipoamide Dehydrogenase (LAD) (EC 1.8.1.4) which is the third catalytic subunit (E3) in three enzymatic complexes in the mitochondrial matrix, crucial for the metabolism of sugars and amino acids - the α-keto acid dehydrogenase complexes. This includes the pyruvate dehydrogenase complex (PDHC), α-ketoglutarate dehydrogenase complex (KGDHC), and branched-chain keto-acid dehydrogenase complex (BCKDHC). LAD is also a component (L-protein) of the glycine cleavage system in mitochondria (Chinnery, 2003; Brautigam, 2005).

Defects in any of the numerous mitochondrial biochemical pathways can cause mitochondrial disease. One such mitochondrial disease is Lipoamide Dehydrogenase (LAD) deficiency (Elpeleg 1997). LAD is a flavoprotein disulfide oxidoreductase that catalyzes the reversible re-oxidation of protein-bound dihydrolipoyl moiety, with NAD⁺ serving as its final electron acceptor (Vettakkorumakankav 1996). The LAD precursor is synthesized with an N-terminal 35AA MTS sequence. A significant number of patients have been diagnosed with LAD deficiency (Berger 1996, Elpeleg 1997, Shaag 1999). This autosomal recessive inherited disorder results in extensive metabolic disturbances due to the reduction in activities of all three α-ketoacid dehydrogenase complexes. Symptoms include lactic acidemia, Krebs cycle dysfunction, and impaired branched-chain amino acid degradation. The clinical course of LAD deficiency may present in infancy with a neurological disease of varying severity or later in life with recurrent episodes of liver failure or myoglobinuria (Shaag, 1999).

The molecular basis of LAD deficiency has been elucidated, and genotype-phenotype correlation is evident (Shaag 1999). Most mutations are associated with the severe neurodegenerative course, e.g. D479V, P488L, K72E, R495G, Y35X, E375K and an in-frame deletion of Gly136 Most patients die in early childhood. In homozygotes for the G229C mutation, a common mutation in Ashkenazi Jews (carrier rate ∼ 1:94), the central nervous system is spared between episodes. Compound heterozygosity for the mutations G229C and Y35X is associated with episodic liver disease and moderate neurological involvement (Shaag, 1999).

### Complex I deficiency

Complex I is the major entry point of electrons into the mitochondrial respiratory chain and contributes to the establishment of a proton gradient required for ATP synthesis. Complex I is the most complicated of the respiratory chain complexes, containing 45 different subunits in mammals, forming a complex of ∼1 MDa. Of the Complex I subunits, seven are encoded by mitochondrial DNA (mtDNA) whereas the remainder are encoded by nuclear genes, translated in the cytosol and imported into the organelle via the outer and inner membrane translocases. Complex I has a bipartite L-shaped configuration consisting of a peripheral matrix arm and a membrane arm. Isolated Complex I deficiency is the most common of the mitochondrial metabolic disorders, accounting for one-third of all cases of respiratory chain deficiency. Mutations in mtDNA genes are detected in only 20% of the patients, suggesting that most patients with isolated complex I deficiencies bear mutations in nuclear genes encoding Complex I subunits.

Currently, there is no cure for genetic mitochondrial metabolic disorders. Treatment is mostly palliative.

### Enzyme Replacement Therapy

Enzyme Replacement Therapy (ERT) is a therapeutic approach for metabolic disorders whereby the deficient or absent enzyme is artificially manufactured, purified and given intravenously to the patient on a regular basis. ERT has been accepted as the treatment of choice for metabolic lysosomal storage diseases, including Gaucher disease (Sly WS. Enzyme replacement therapy: from concept to clinical practice. Acta Paediatr, Suppl 91(439):71-8, 2002), Fabry disease (Desnick RJ et al, Fabry disease: clinical spectrum and evidence-based enzyme replacement therapy. Nephrol Ther, Suppl 2:S172-85, 2006), and attenuated variants of mucopolysaccaridoses (MPS 1)

Scarpa M et al, Mucopolysaccharidosis VI: the Italian experience. Eur J Pediatr. Jan 7, 2009). However, ERT has never been shown, believed, or even suggested to useful in treating disorders involving enzymatic components of multi-component enzyme complexes such as the PDHC. Moreover, the inability of the intravenously administered enzymes to penetrate the blood-brain barrier severely limits the application of this approach for treatment of other metabolic disorders involving the central nervous system (Brady, 2004).

One approach for delivering proteins into cells is fusion with protein transduction domains (PTDs). Most PTDs are cationic peptides (11-34 amino acids) that interact with the negatively charged phospholipids and carbohydrate components of the cell membrane Futaki 2001. PTDs enable passage of a protein through cell membranes in a fashion not clearly understood, but believed to be via neither phagocytosis nor receptor-mediated, clathrin-pit endocytosis. The most well-known and used PTD is HIV-1 TransActivator of Transcription (TAT) peptide. TAT peptide is an 11-amino-acid (residues 47-57) arginine-and lysine-rich portion of the HIV-1 Tat protein having the sequence set forth in SEQ ID NO: 10 (Kuppuswamy 1989). TAT-fusion proteins can be introduced into cultured cells, intact tissue, and live tissues and cross the blood-brain barrier (BBB) when injected into mice (Futaki 2001; Del Gaizo 2003a, Del Gaizo 2003b.

TAT fusion proteins traverse also mitochondrial membranes. When an MTS is present, a Green Fluorescent Protein (GFP) is retained within the mitochondrial matrix over time and persists within tissues of injected mice for several days (Del Gaizo, V et al. Targeting proteins to mitochondria using TAT. Mol. Genet. Metab 80: 170-180, 2003; Del Gaizo, V et al. A novel TAT-mitochondrial signal sequence fusion protein is processed, stays in mitochondria, and crosses the placenta. Mol. Ther. 7: 720-730, 2003). WO 05/042560 to Payne discloses in addition use of TAT to target frataxin to mitochondria, but the translocated frataxin is not shown to have any functionality.

US 20060211647 to Khan discloses use of a PTD to introduce GFP and transcription factor A (TFAM) into mitochondria.

WO 05/001062 to Khan discloses targeting of nucleic acids to mitochondria using a vector comprising a protein transduction domain, Arg11 (SEQ ID NO: 38) to the head protein of a vector and delivery of GFP and Red Fluorescent Protein using same.

Rapoport M. et al., Journal of Inherited Metabolic Diseases, August 2006, Vol. 29, no. Suppl. 1, p. 15, describes an ERT for mitochondrial diseases such as LAD deficiency, using a fusion protein comprising LAD and the HIV TAT peptide. The LAD delivered into cells and their mitochondira replaces the mutated endogenous enzyme. The TAT-LAD is naturally incorporated into α-keto acid dehydrogenase complexes.

Angdisen J. et al., Current Drug Targets. Immune, Endocrine and Metabolic disorders, Mar 2005, Vol. 5, no. 1, pages 27-40 relates to mitochondrial trifunctional protein (MTP) defects and describes a construct of green fluorescent protein (GFP) fused to TAT and MTS (mitochondrial targeting sequence), which localized into cells mitochndria.

Toro A. et al., The journal of clinical Investigation, October 2006, Vol. 116, no. 10, pages 2717, describes TAT-mediated intracellular delivery of the cytoplasmic enzyme purine nucleoside phosphorylase, to various tissues/organs.

Khan S.M. et al., Journal of Bioenergetics and Biomembranes, august 2004, Vol. 36, no. 4, pages 387-393 relates to gene therapy and discloses transduction technology (protofection) that allows insertion and expression of human mitochondrial genes into mitochondria of living cells.

None of the above references disclose or suggest that sufficient quantities of an enzyme attached to a PTD can, after crossing both the cellular and mitochondrial membranes, retain not only enzymatic activity but proper conformation to form a functional component of a multi-component enzyme complex or replace missing physiological function in a mitochondria metabolic disorder. Furthermore, none of the above references discloses or suggests that such a strategy would work despite the presence of a mutated enzyme (missense) enzyme in the complex, which would be expected to block integration of significant quantities of the functional enzyme.

### SUMMARY OF THE INVENTION

The present invention provides a fusion protein for use in a method of treatment of a mitochondrial disorder in a subject, said fusion protein comprising a protein transduction domain (PTD) fused to a functional component of a mitochondrial enzyme and a mitochondria targeting sequence (MTS), wherein said MTS is situated between said PTD and said functional component and wherein said MTS is an MTS of another mitochondrial enzyme that is encoded by a nuclear gene, wherein said mitochondrial enzyme is an enzyme of a mitochondrial multi-component enzyme complex, and wherein said protein transduction domain is a TAT peptide, wherein said treatment comprises administering said fusion protein to said subject and is a continuous prolonged treatment for a chronic disease or comprises a single or few time administrations for treatment of an acute condition.

Disclosed is a pharmaceutical composition comprising a pharmaceutically acceptable carrier and as an active ingredient a fusion protein comprising a protein transduction domain fused to a functional component of a mitochondrial enzyme.

The pharmaceutical composition is for the treatment of a mitochondrial disorder.

Also disclosed is the use of a fusion protein comprising a protein transduction domain fused to a functional component of a mitochondrial enzyme, for the preparation of a medicament for the treatment of a mitochondrial disorder.

The fusion protein comprises a mitochondria targeting sequence (MTS). The MTS is present between the protein transduction domain and the functional component of the mitochondrial enzyme.

Also disclosed is a method for the treatment of a mitochondrial disorder, comprising administering to a subject in need of such treatment a therapeutically effective amount of a fusion protein comprising a protein transduction domain fused to a functional component of a mitochondrial enzyme.

The term *"**fusion protein**"* in the context of the invention concerns a sequence of amino acids, predominantly (but not necessarily) connected to each other by peptidic bonds, wherein a part of the sequence is derived (i.e. has sequence similarity to sequences) from one origin (native or synthetic) and another part of the sequence is derived from one or more other origin. This term refers to the origin of the sequences, as in practice when the protein is prepared by recombinant techniques there is no distinction between the "fused" parts.

The term *"**fused**"* in accordance with the fusion protein of the invention, refers to the fact that the sequences of the two origins, preferably also the sequences of the mitochondrial translocation domain, MTS and mitochondrial enzyme, are linked to each other by covalent bonds. The fusion may be by chemical conjugation such as by using state of the art methodologies used for conjugating peptides. However, in accordance with a preferred embodiment, the fusion is preferably by recombinant techniques, i.e. by construction a nucleic acid sequence coding for the whole of the fusion protein (coding for both sections) so that essentially all the bonds are peptidic bonds. Such recombinantly, all peptidic bonds-containing fusion proteins have the advantage that the product features greater homogeneity as compared to chemically conjugated chimeric molecules.

The term *"**protein transduction domain (PTD)**"* refers to any amino acid sequence capable of causing the transport of a peptide, sequence, or compound attached to it through cellular membranes independently of receptor-mediated entry. In particular, it is a sequence that can cause the transport through both the cytoplasmic membrane and the mitochondrial membrane. These are cationic peptides characterized by being heavily positively charged; rich in positive amino acids such as arginine or lysine.

Typically these domains are cationic peptides having a length of 11-34 amino acids.

The PTD of the fusion protein for use of the present invention is a Trans-Activator of Transcription (TAT) peptide. A non-limiting example is the domain from the HIV-1 TAT protein (SEQ ID NO: 10).

The term *"**mitochondrial enzyme**"* refers to an enzyme that is essential for a biological activity of mitochondria. The term *"**mitochondrial enzyme complex**"* refers to an enzyme that forms a complex with other enzymes, forming a complex that is essential for a biological activity of mitochondria. Typically these are enzymes or complexes of enzymes which, when lacking or mutated in at least one subunit, causes a mitochondrial disorder.

A specific preferred example is Lipoamide Dehydrogenase (LAD), which is a flavoprotein disulfide oxidoreductase that catalyzes the reversible re-oxidation of protein-bound dihydrolipoyl moiety, with NAD⁺ serving as its final electron acceptor.

"LAD" or dihydrolipoamide dehydrogenase, as used herein, refers to a gene also known as DLD, tcag7.39, DLDH, E3, GCSL, LAD, PHE3, and having GenBank Accession No. NG_008045 and EC number="1.8.1.4". A representative amino acid sequence of LAD is set forth in SEQ ID NO: 16 (GenBank Accession No. NP_000099).

In other embodiment, the mutant enzyme whose activity is supplied by a fusion protein for use of the present invention is selected from the group consisting of 2-oxoisovalerate dehydrogenase alpha subunit (Branched-Chain Keto Acid Dehydrogenase E1α) (NCBI Protein Database Accession No. P12694; OMIM:248600), 2-oxoisovalerate dehydrogenase beta subunit (Branched-Chain Keto Acid Dehydrogenase E1β; P21953), Acyl-CoA dehydrogenase, medium-chain specific (P11310; OMIM:201450), Acyl-CoA dehydrogenase, very-long-chain specific (P49748; OMIM:201475), Trifunctional enzyme alpha subunit (Long-chain 3 hyroxyacyl CoA Dehydrogense or LCHAD) (P40939; OMIM:609015) (HADHA), Trifunctional enzyme beta subunit (Hydroxyacyl-CoA Dehydrogenase/3-Ketoacyl-CoA Thiolase/Enoyl-CoA Hydratase (P55084) (HADHB)), Pyruvate dehydrogenase E1 component beta subunit (P11177; OMIM:208800), and Pyruvate dehydrogenase E1 component alpha subunit (P08559; OMIM:312170).

Each enzyme represents a separate embodiment of the fusion protein for use of the present invention.

The term *"**functional component**"* refers to the fact that the enzyme, as described above, has an enzymatic activity when present in the mitochondria as a part of an enzymatic complex (with other enzymes, co-factors, or proteins) . In one embodiment, the functional component is the full sequence of the enzyme. In another embodiment, the functional component is a domain (fragment) sufficient to carry out the enzymatic activity of the enzyme, either alone or as part of a complex, as appropriate. In another embodiment, the functional component is a mutated derivative wherein one or more of the native amino acid residues has been deleted, replaced or modified while still maintaining the enzymatic functionally of the component (alone or as part of a complex). This term also refers to precursors of the enzymes which in the cell or in the mitochondrial are converted into a functional enzyme or are assembled to form a functional enzymatic complex. In another embodiment, the term refers to any fragment of the enzyme comprising the catalytic domain thereof, wherein the conformation of the fragment under physiological conditions is such that the enzymatic activity of the catalytic domain is maintained. Each possibility represents a separate embodiment of the present invention.

The term *"**multi-component enzyme complex**"* refers to a group of at least two different enzymes assembled together in a specific ratio that functions in a coordinated fashion to catalyze a series of reactions. The function of a multi-component enzyme complex is dependent on its structure; thus, the enzymes that compose the complex must physically fit together in the proper configuration in order to efficiently catalyze the series of reactions. Non-limiting examples of mitochondrial multi-component enzyme complexes are pyruvate dehydrogenase complex (PDHC), α-ketoglutarate dehydrogenase complex (KGDHC), and branched-chain keto-acid dehydrogenase complex (BCKDHC) (those listed thus far contain LAD), the complexes of the respiratory chain, and those involved in fatty acid β-oxidation and the urea cycle. The complexes of the respiratory chain are complex I (NADH-ubiquinone oxidoreductase), complex II (succinate-ubiquinone oxidoreductase), complex III (ubiquinol-ferricytochrome c oxidoreductase), complex IV (cytochrome c oxidoreductase), and complex V (F1F0 ATPase).

Each multi-component enzyme complex represents a separate embodiment of the fusion protein for use of the present invention.

The term *"**mitochondrial targeting sequence (MTS)**"* refers to any amino acid sequence capable of causing the transport of a peptide, sequence, or compound attached to it into the mitochondria. In another embodiment, the MTS is a human MTS. In another embodiment, the MTS is from another species. Non-limiting examples of such sequences are the human LAD MTS (SEQ ID NO: 39), the MTS of the C6ORF66 gene product (SEQ ID NO: 9), and the MTS's from human mitochondrial malate dehydrogenase (SEQ ID NO: 40), OGG1 (SEQ ID NO: 49) and GLUD2 (SEQ ID NO: 50). Additional non-limiting examples of MTS sequences are the natural MTS of each individual mitochondrial protein that is encoded by the nuclear DNA, translated (produced) in the cytoplasm and transported into the mitochondria. The various MTS may be exchangeable for each mitochondrial enzyme among themselves. Each possibility represents a separate embodiment of the fusion protein for use of the present invention.

It should be noted that each mitochondrial enzyme that is produced in the cytoplasm and transported into the mitochondria is produced as a precursor enzyme carrying its natural MTS, so that using the precursor mitochondrial enzyme already has its MTS; however, this naturally occurring sequence in the precursor enzyme can be exchanged with any other known MTS, mainly to increase translocation efficacy.

The term *"**mitochondrial disorder**"* in the context of the invention refers to a group of systemic diseases caused by inherited or acquired damage to the mitochondria causing an energy shortage within those areas of the body that consume large amounts of energy such as the liver, muscles, brain, and the heart. The result is often liver failure, muscle weakness, fatigue, and problems with the heart, eyes, and various other systems. In certain preferred embodiments, the mitochondrial disorder is LAD deficiency.

In certain other preferred embodiments, the mitochondrial metabolic disorder is Complex I deficiency (OMIM:252010). Complex I deficiency can be caused by a mutation in any of the subunits thereof. In another embodiment, the Complex I deficiency is caused by a mutation in a gene selected from the group consisting of NDUFV1 (OMIM:161015), NDUFV2 (OMIM:600532), NDUFS1 (OMIM:157655), NDUFS2 (OMIM:602985), NDUFS3 (OMIM:603846), NDUFS4 (OMIM:602694), NDUFS6 (OMIM:603848), NDUFS7 (OMIM:601825), NDUFS8 (OMIM:602141), and NDUFA2 (OMIM:602137).

In another embodiment, the mitochondrial metabolic disorder is Complex IV deficiency (cytochrome c oxidase; OMIM:220110). Complex IV deficiency can be caused by a mutation in any of the subunits thereof. In another embodiment, the Complex IV deficiency is caused by a mutation in a gene selected from the group consisting of MTCO1 (OMIM:516030), MTCO2 (OMIM:516040), MTCO3 (OMIM:516050), COX10 (OMIM:602125), COX6B1 (OMIM:124089), SCO1 (OMIM:603644), FASTKD2 (OMIM:612322), and SCO2 (OMIM:604272).

In other embodiments, the mitochondrial disorder is caused by or associated with a missense mutation in the enzyme whose activity is being replaced. As provided herein, compositions of the present invention exhibit the surprising ability to complement missense mutations, despite the presence of the mutated protein in multi-component enzyme complexes.

In other embodiments, the mitochondrial disorder is a neurodegenerative disease. As provided herein, fusion proteins for use of the present invention exhibit the ability to traverse the blood-brain barrier (BBB). In this embodiment, a PTD capable of traversing the BBB will be selected.

In other embodiments, the mitochondrial disorder is selected from the group consisting of encephalopathy and liver failure that is accompanied by stormy lactic acidosis, hyperammonemia and coagulopathy.

In other embodiments, the mitochondrial disorder is selected from the group consisting of Ornithine Transcarbamylase deficiency (hyperammonemia) (OTCD), Carnitine O-palmitoyltransferase II deficiency (CPT2), Fumarase deficiency, Cytochrome c oxidase deficiency associated with Leigh syndrome, Maple Syrup Urine Disease (MSUD), Medium-Chain Acyl-CoA Dehydrogenase deficiency (MCAD), Acyl-CoA Dehydrogenase Very Long-Chain deficiency (LCAD), Trifunctional Protein deficiency, Progressive External Ophthalmoplegia with Mitochondrial DNA Deletions (POLG), DGUOK, TK2, Pyruvate Decarboxylase deficiency, and Leigh Syndrome (LS).In another embodiment, the mitochondrial metabolic disorder is selected from the group consisting of Alpers Disease; Barth syndrome; β-oxidation defects; carnitine-acyl-camitine deficiency; carnitine deficiency; co-enzyme Q10 deficiency; Complex II deficiency (OMIM:252011), Complex III deficiency (OMIM:124000), Complex V deficiency (OMIM:604273), LHON-Leber Hereditary Optic Neuropathy; MM-Mitochondrial Myopathy; LIMM-Lethal Infantile Mitochondrial Myopathy; MMC-Maternal Myopathy and Cardiomyopathy; NARP-Neurogenic muscle weakness, Ataxia, and Retinitis Pigmentosa; Leigh Disease; FICP-Fatal Infantile Cardiomyopathy Plus, a MELAS-associated cardiomyopathy; MELAS-Mitochondrial Encephalomyopathy with Lactic Acidosis and Strokelike episodes; LDYT-Leber's hereditary optic neuropathy and Dystonia; MERRF-Myoclonic Epilepsy and Ragged Red Muscle Fibers; MHCM-Maternally inherited Hypertrophic CardioMyopathy; CPEO-Chronic Progressive External Ophthalmoplegia; KSS-Kearns Sayre Syndrome; DM-Diabetes Mellitus; DMDF Diabetes Mellitus + Deafness; CIPO-Chronic Intestinal Pseudoobstruction with myopathy and Ophthalmoplegia; DEAF-Maternally inherited DEAFness; PEM-Progressive encephalopathy; SNHL-SensoriNeural Hearing Loss; Encephalomyopathy; Mitochondrial cytopathy; DEMCHO-Dementia and Chorea; AMDF-Ataxia, Myoclonus; ESOC Epilepsy; Optic atrophy; FBSN Familial Bilateral Striatal Necrosis; FSGS Focal Segmental Glomerulosclerosis; LIMM Lethal Infantile Mitochondrial Myopathy; MDM Myopathy and Diabetes Mellitus; MEPR Myoclonic Epilepsy and Psychomotor Regression; MERME MERRF/MELAS overlap disease; MHCM Maternally Inherited Hypertrophic CardioMyopathy; MICM Maternally Inherited Cardiomyopathy; MILS Maternally Inherited Leigh Syndrome; Mitochondrial Encephalocardiomyopathy; Multisystem Mitochondrial Disorder (myopathy, encephalopathy, blindness, hearing loss, peripheral neuropathy); NAION Nonarteritic Anterior Ischemic Optic Neuropathy; PEM Progressive Encephalopathy; PME Progressive Myoclonus Epilepsy; RTT Rett Syndrome; SIDS Sudden Infant Death Syndrome; and MIDD Maternally Inherited Diabetes and Deafness.

Mitochondrial disorders are inherited or acquired disorders, although rarely they can be the result of a spontaneous mutation in early development of the embryo. The two most common inheritance patterns of mitochondrial cytopathies are Mendelian and Maternal. Some representative examples of mitochondrial diseases are depicted in the table below.

| **Disease** | **Protein affected** | **OMIM** |
|---|---|---|
| Ornithine Transcarbamylase deficiency (hyperammonemia) (OTCD | Ornithine Transcarbamylase (P00480) | 311250 |
| Carnitine O-palmitoyltransferase II deficiency (CPT2) | Carnitine O-palmitoyltransferase II (P23786) | 255110 |
| Fumarase deficiency | Fumarate hydratase (P07954) | 606812 |
| Cytochrome c oxidase deficiency associated with Leigh syndrome | Surfeit locus protein 1(SURF1) (Q15526) | 220110 |
| Maple Syrup Urine Disease (MSUD) | 1. 2-oxoisovalerate dehydrogenase alpha subunit (Branched-Chain Keto Acid Dehydrogenase E1α) (P12694) | 248600 |
| | 2. 2-oxoisovalerate dehydrogenase beta subunit (Branched-Chain Keto Acid Dehydrogenase E1β)(P21953) | |
| Medium-Chain Acyl-CoA Dehydrogenase deficiency (MCAD) | Acyl-CoA dehydrogenase, medium-chain specific (P1310) | 201450 |
| Acyl-CoA Dehydrogenase Very Long-Chain deficiency (LCAD) | Acyl-CoA dehydrogenase, very-long-chain specific (P49748) | 201475 |
| Trifunctional Protein deficiency | 1. Trifunctional enzyme alpha subunit (Long-chain 3 hyroxyacyl CoA Dehydrogense (LCHAD)(P40939) (HADHA). | 609015 |
| | 2. Trifunctional enzyme beta subunit, [Hydroxyacyl-CoA Dehydrogenase/3-Ketoacyl-CoA Thiolase/Enoyl-CoA Hydratase, (P55084)(HADHB) | |
| Progressive External Ophthalmoplegia with Mitochondrial DNA Deletions (POLG) | DNA polymerase gamma subunit 1 (P54098) | 157640 |
| DGUOK | Deoxyguanosine kinase | 601465 |
| TK2 | Thymidine kinase-2 | 188250 |
| Pyruvate Decarboxylase deficiency | Pyruvate dehydrogenase E1 component beta subunit (P11177) | 208800 |
| | Pyruvate dehydrogenase E1 component alpha subunit (P08559) | 312170 |
| Leigh Syndrome (LS) | Leigh syndrome may be a feature of a deficiency of any of the mitochondrial respiratory chain complexes: I (OMIM:252010), II (OMIM:252011), III (OMIM:124000), IV (cytochrome c oxidase; OMIM:220110), or V (OMIM:604273). | |

Each mitochondrial disease represents a separate embodiment of the fusion protein for use of the present invention. The term *"**treatment**"* in the context of the intention does not refers to complete curing of the diseases, as it does not change the mutated genetics causing the disease. This term refers to alleviating at least one of the undesired symptoms associated with the disease, improving the quality of life of the subject, decreasing disease-caused mortality, or (if the treatment in administered early enough)- preventing the full manifestation of the mitochondrial disorder before it occurs, mainly to organs and tissues that have a high energy demand. The treatment may be a continuous prolonged treatment for a chronic disease or a single, or few time administrations for the treatment of an acute condition such as encephalopathy and liver failure that is accompanied by stormy lactic acidosis, hyperammonemia and coagulopathy.

The inventors of the present invention used the "LAD Deficiency" disease as a model; however, the scope of this invention is not restricted to this disease.

In accordance with a specific example of the invention, the human precursor LAD enzyme was fused to a delivery moiety (TAT), which led this enzyme into cells and their mitochondria, thus substituting for the mutated endogenous enzyme.

To test this approach, the TAT-LAD fusion protein was constructed and highly purified. It was shown that TAT-LAD is able to enter patients' cells and their mitochondria while augmenting LAD activity. Furthermore, it was shown that TAT-LAD is able to substitute for the mutated LAD enzyme within the mitochondrial enzyme complex pyruvate dehydrogenase complex (PDHC), thus restoring its activity to nearly normal levels.

### BRIEF DESCRIPTION OF THE DRAWINGS

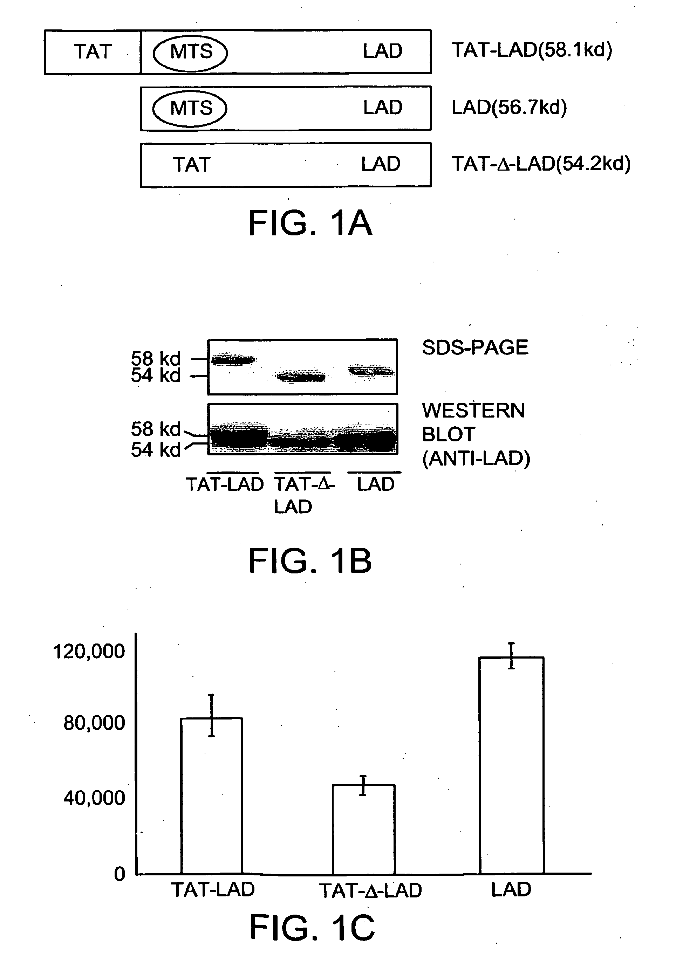

**Figure 1****.** Schematic representation of TAT-LAD and LAD fusion proteins, their expression and purification. **A.** Schematic representation of TAT-LAD fusion protein and the control proteins-TAT-Δ-LAD (lacking the MTS moiety) and LAD (lacking the TAT moiety). **B.** Sodium dodecyl sulfate polyacrylamide gel electrophoresis (SDS-PAGE) and Western blot using anti-LAD antibody analysis of purified TAT-LAD, TAT-Δ-LAD, and LAD fusion proteins. Proteins were purified using affinity chromatography. **C.** Enzymatic activity of purified TAT-LAD, TAT-Δ-LAD, and LAD fusion proteins. LAD activity values (nmol/min/mg) presented are mean values ± SD of three separate enzymatic assays, each carried out in triplicate. LAD: lipoamide dehydrogenase; MTS: mitochondrial targeting sequence; TAT: transactivator of transcription peptide.

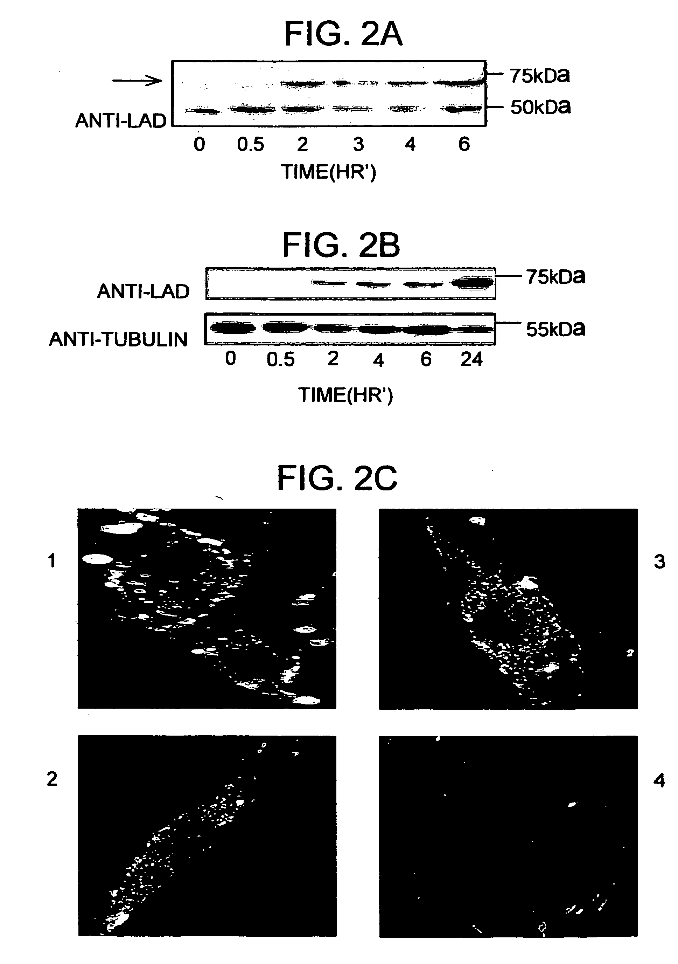

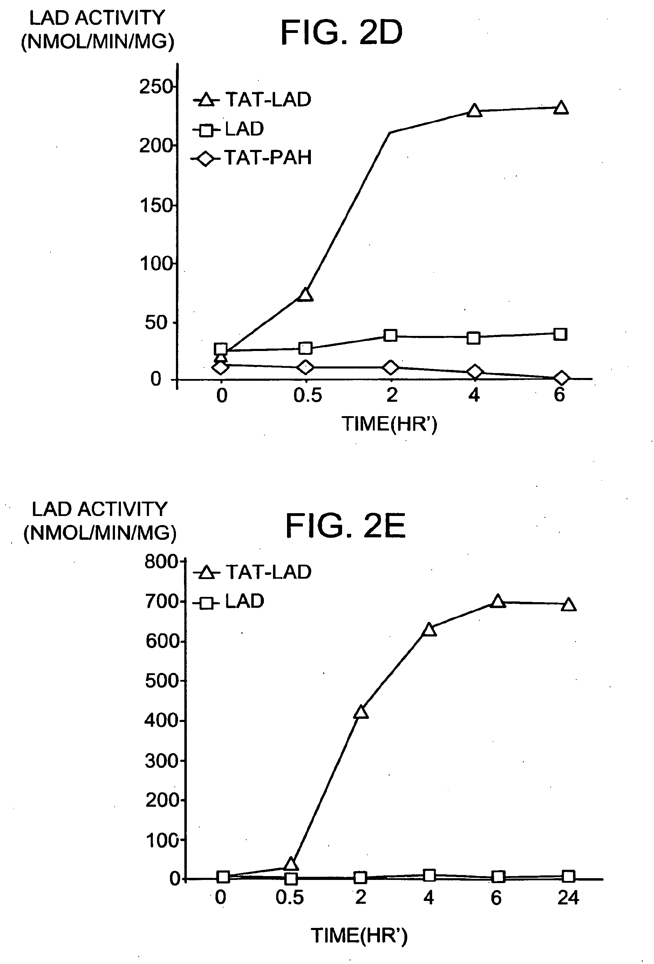

**Figure 2****.** Delivery of TAT-LAD into G229C/Y35X and E375K patients' cells. **A.** Western blot analysis of whole-cell protein extracts from G229C/Y35X-treated cells using antibodies against LAD (1:1000). TAT-LAD fusion protein (arrow) and endogenous mutated LAD correspond to M.W. of 58kDa and 50kDa, respectively, as expected. **B.** Western blot analysis of whole-cell protein extracts from E375K-treated cells, using antibodies against LAD (1:1000) and α-tubulin (1:10,000). Anti-Tubulin served as an internal control for protein loading. **C.** Fluorescence microscopy analysis of G229C/Y35X cells treated with FITC-labeled TAT-LAD (panels 1-3) and LAD (panel 4) (0.1µg/µl final concentration) for 30 min (panel 1), 2hrs (panel 2) and 4hrs (panel 3). **D-E.** LAD activity in treated G229C/Y35X (**D**) and E375K (E) cells. Cells were treated with TAT-LAD, TAT-PAH or LAD protein (0.075-0.1µg/µl, final concentration) for different time periods. LAD activity was analyzed in whole-cell protein extracts by enzymatic activity assay. Activity assays were conducted at least three times. Values are presented as the mean ± s.e.m. (left panel) or depict typical results (right panel). The activity values are presented as nmol/min/mg protein.

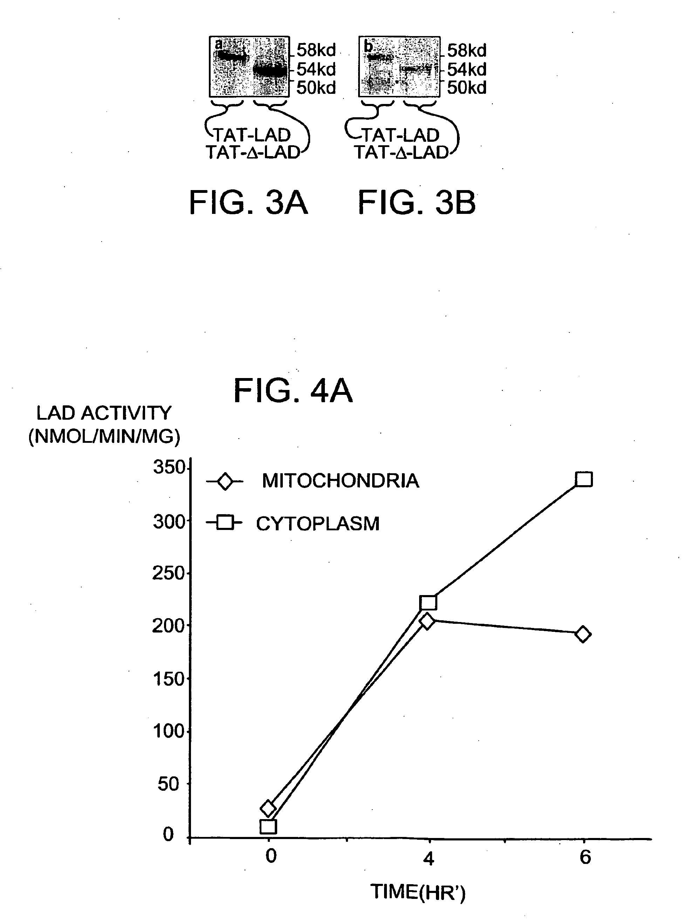

**Figure 3****.** Fate of TAT-LAD and TAT-Δ-LAD within isolated mitochondria. (**A**) Radioactive-labeled TAT-LAD and TAT-Δ-LAD were expressed *in vitro* and analyzed using SDS-PAGE autoradiography, matching their expected molecular sizes, 58 and 54 kd, respectively. (**B**) Mitochondria isolated from cells were incubated for 30 min. with the radio-labeled proteins. Mitochondria were then washed, treated with proteinase K, and analyzed using SDS-PAGE autoradiography. Asterisk marks 50 kd band of processed TAT-LAD fusion protein.

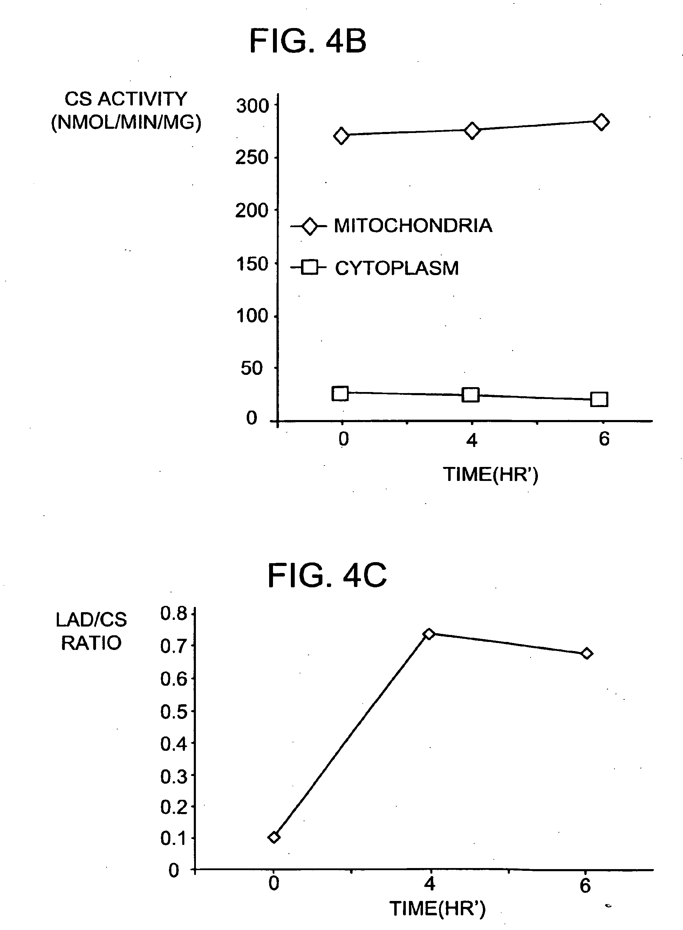

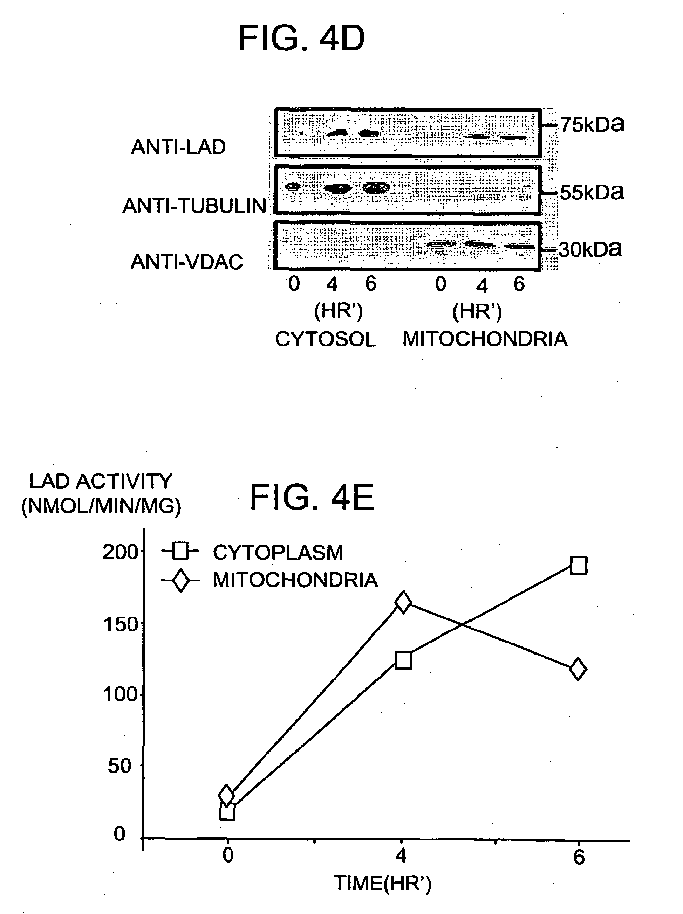

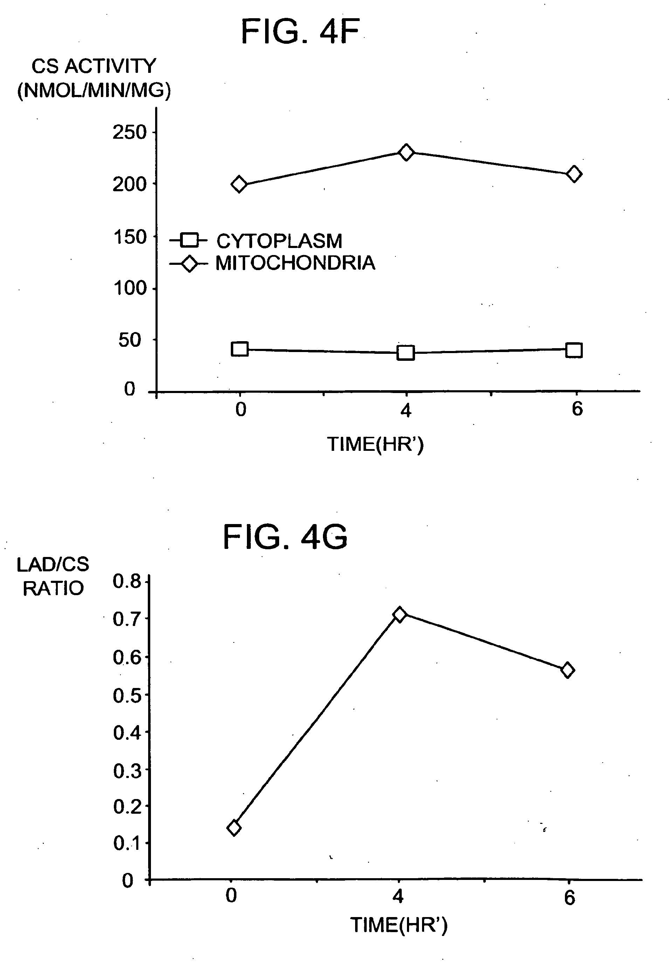

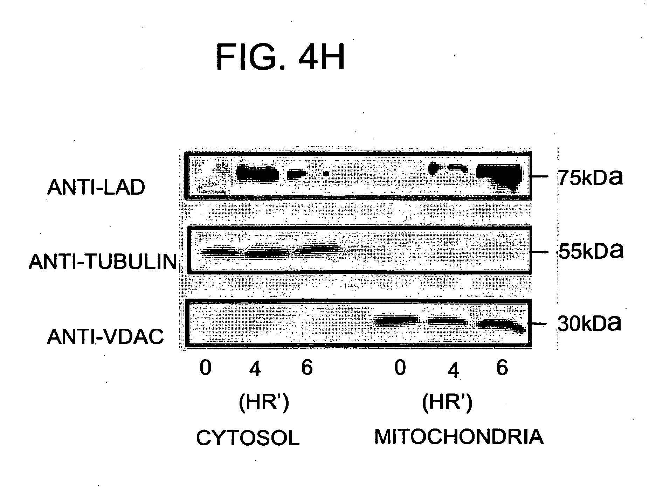

**Figure 4****.** Delivery of TAT-LAD into mitochondria of G229C/Y35X (A-D) and D479V (**E-H**) patients' cells. Cells were treated with the fusion protein (0.1µg/µl final concentration) for 4-6hrs. Sub-cellular fractions (cytosolic and mitochondrial) were obtained by differential centrifugation. The LAD (**A,E**) and CS (**B,F**) enzymatic activities in the cytosolic and mitochondrial fractions of the treated cells were analyzed and the LAD/CS ratio (**C,G**) in their mitochondrial fraction was calculated. The LAD/CS ratio was almost two-fold higher for TAT-LAD than for TAT-ΔLAD. Activity values are presented as nmol/min/mg protein. **D** and **H.** Western blot analysis of the sub-cellular fractions showing the intra-cellular distribution of TAT-LAD and their purity, using antibodies against LAD (1:1000) and the specific markers VDAC (porin) (1:5000) for the mitochondria and α-tubulin (1:10000) for the cytoplasm. The marker E1α was also used to confirm purity of the mitochondrial fraction.

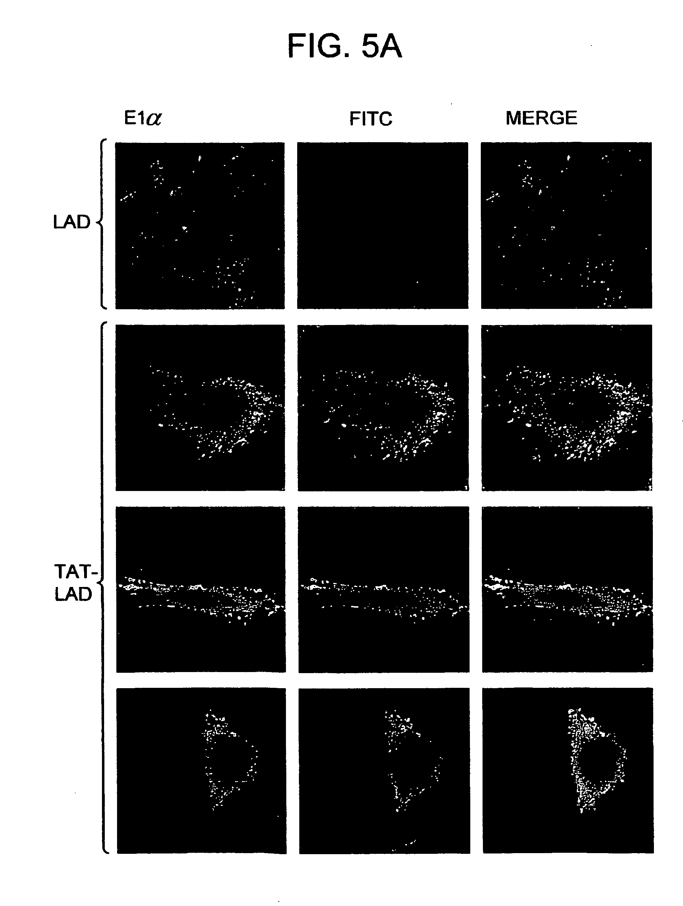

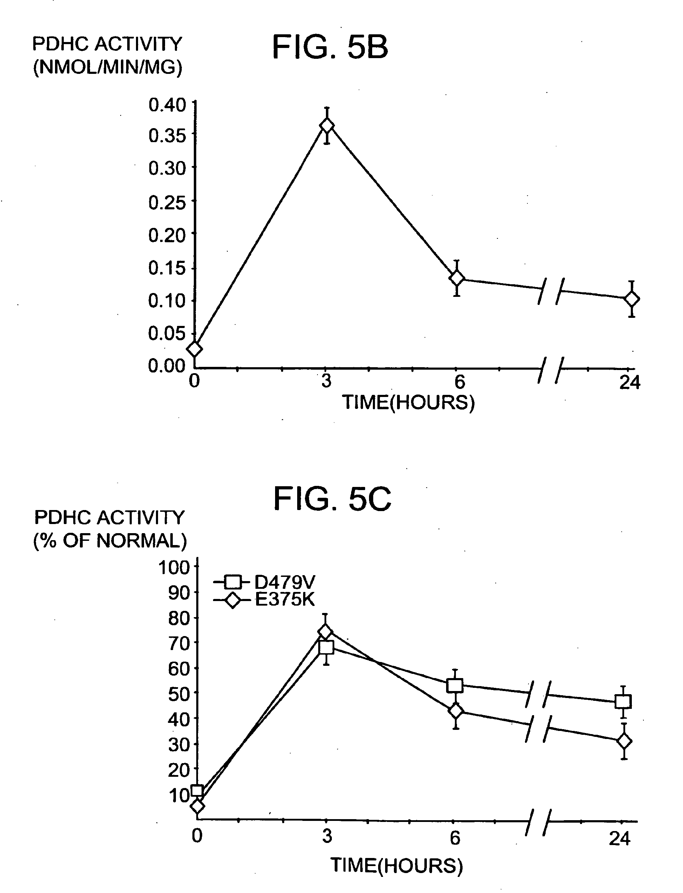

**Figure 5****.** PDHC co-localization and enzymatic activity in TAT-LAD-treated cells from patients. (**A**) D479V cells were treated with fluorescein isothiocyanate (FITC)-labeled TAT-LAD or LAD (green fluorescence, middle column), washed, fixed, permeabilized, and incubated with anti-E1α antibody. The cells were then washed and incubated with anti-mouse Cy5 antibody (red fluorescence, left column). The cells were analyzed for co-localization using confocal microscopy (yellow merge, right column). Original magnifications: 60 (LAD) and 100 (TATLAD). (**B-C**) Cells were incubated with TAT-LAD (0.1 µg/µl, final concentration) for 3, 6, or 24 hours. PDHC activity assays were performed as described in Materials and Methods. (**B**) PDHC activity in treated E375K cells of patients. Activity values are presented as nmol/min/mg protein. (**C**) PDHC activity in treated E375K and D479V cells of patients. Activity values are presented as the percentage of normal PDHC activity measured in healthy fibroblasts in the same experiments. Activity assays were repeated three times. Values presented in **B** and **C** are mean values ± SD. PDHC, pyruvate dehydrogenase complex. Co-localization was also observed in treated G229C/Y35X patients' cells.

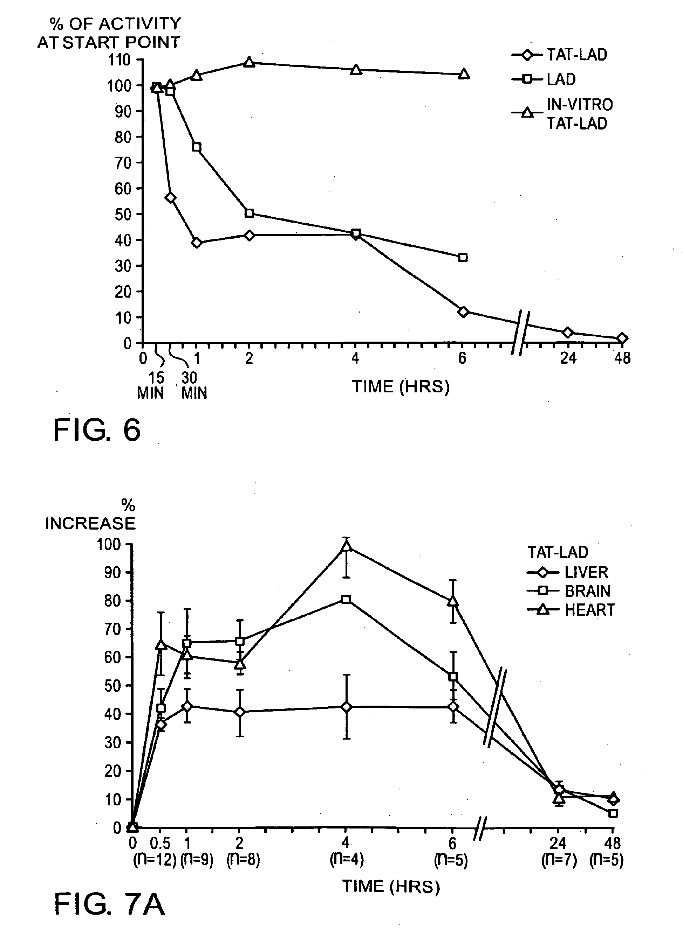

**Figure 6****:** Enzymatic Activity of LAD in plasma of E3 mice injected with TAT-LAD. Behavior and stability of the injected TAT-LAD were followed in the plasma of injected mice by measuring LAD enzymatic activity. Blood samples from E3 injected mice were withdrawn at different time points, and plasma was prepared.

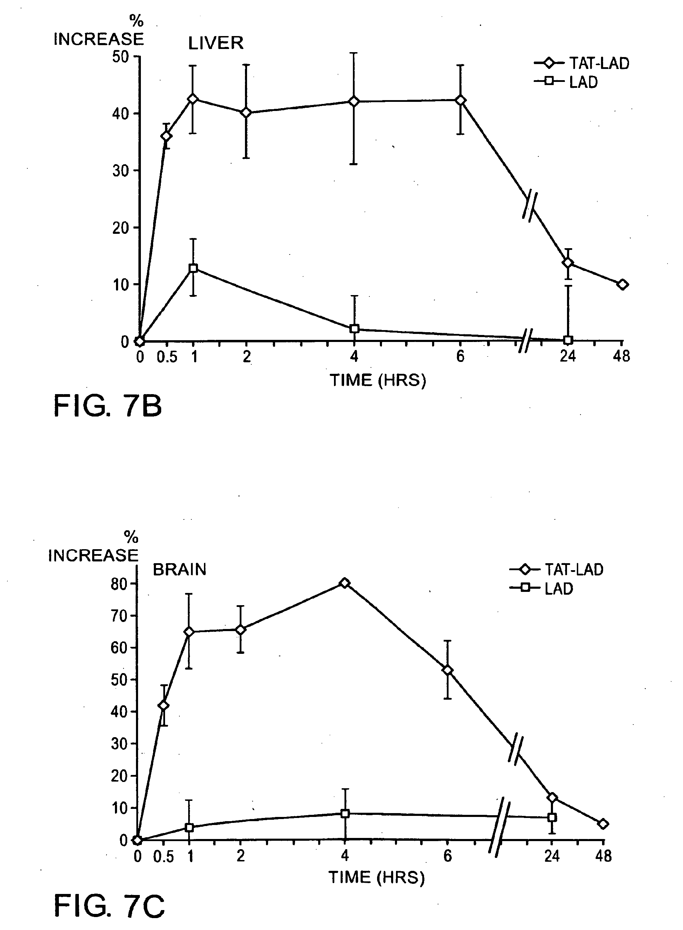

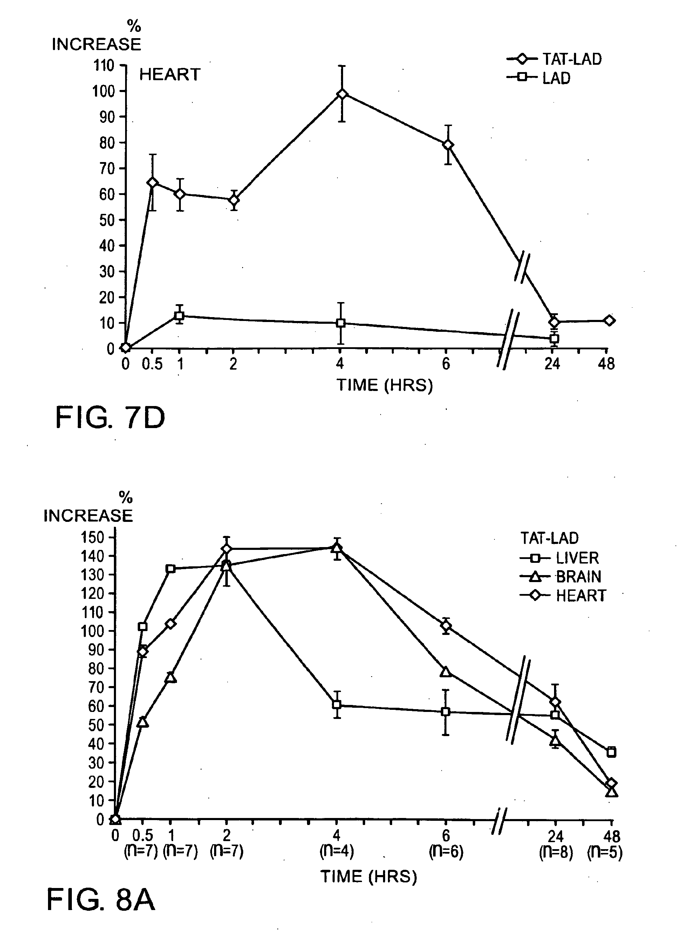

**Figure 7****: A.** TAT-LAD activity in various organs of E3 mice treated with TAT-LAD: Time dependency. LAD activity is presented as percent increase from the basal activity measured in the non-treated (PBS-injected) E3 mice. **B-D.** Effect of TAT-LAD vs. LAD control protein in liver (**B**), brain (**C**), and heart (**D**).

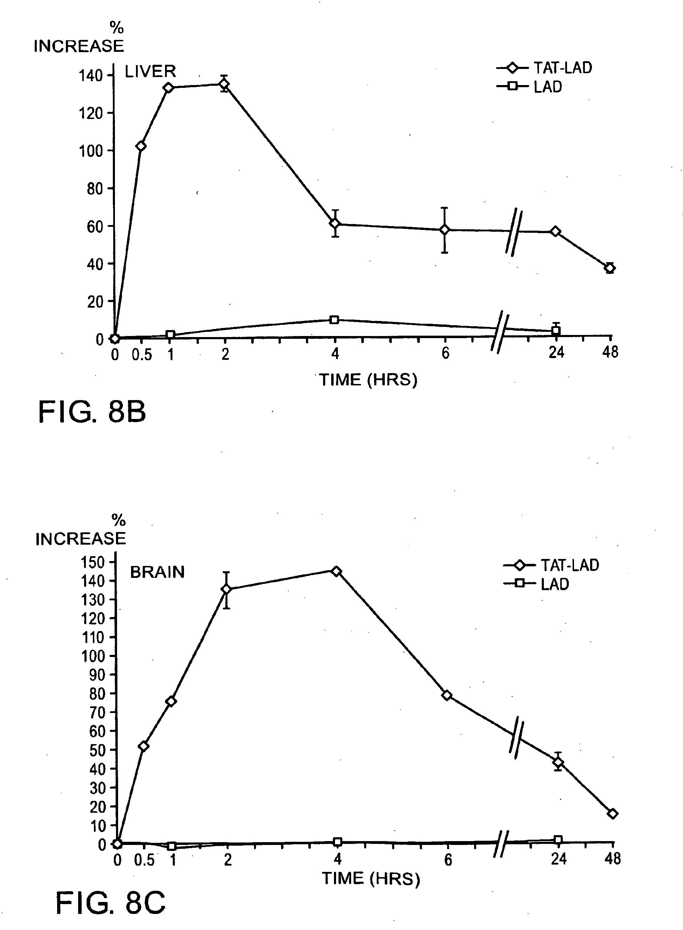

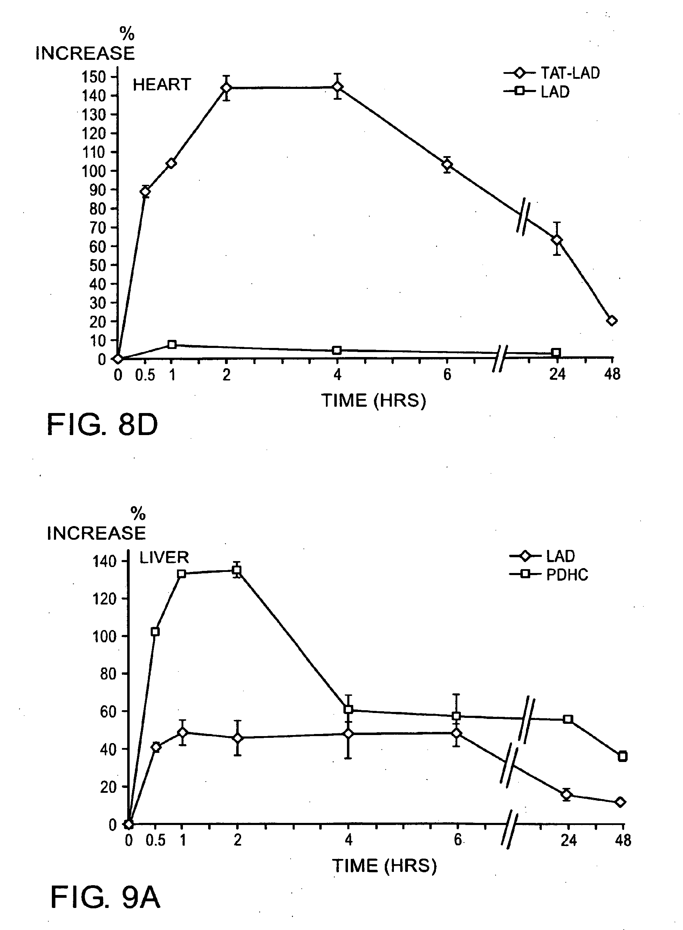

**Figure 8****:** A. PDHC Activity in organs of E3 mice treated with TAT-LAD. Results are presented as the percent increase over basal PDHC activity in the same organ of the non-treated (PBS-injected) E3 mice. **B-D.** Effect of TAT-LAD vs. LAD control protein in liver (**B**), brain (**C**), and heart (**D**).

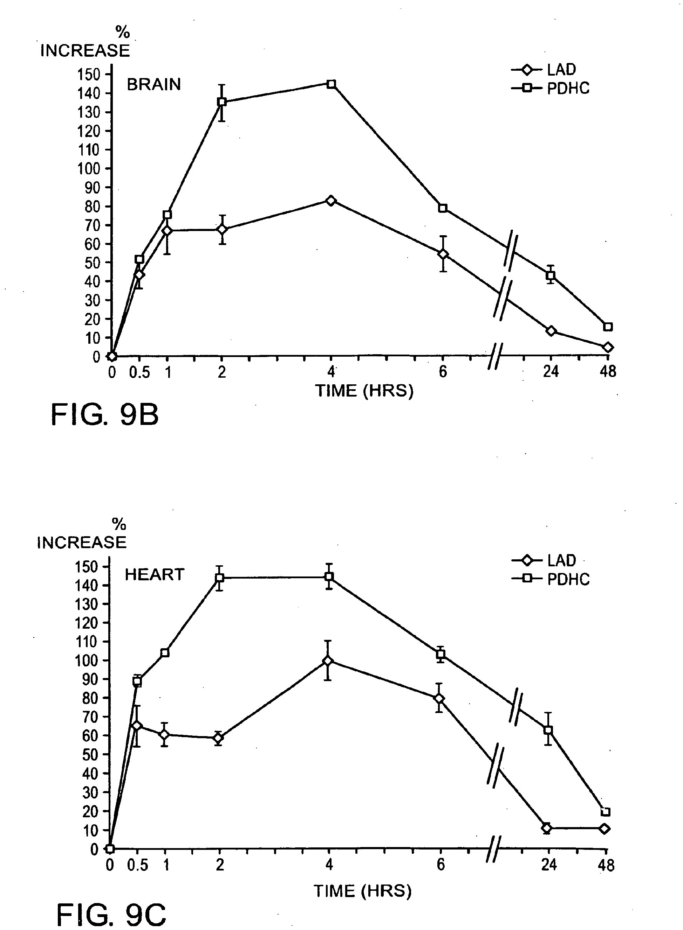

**Figure 9****:** PDHC Activity vs. LAD activity in organs of E3 mice treated with TAT-LAD. **A.** liver. **B.** brain. C. heart.

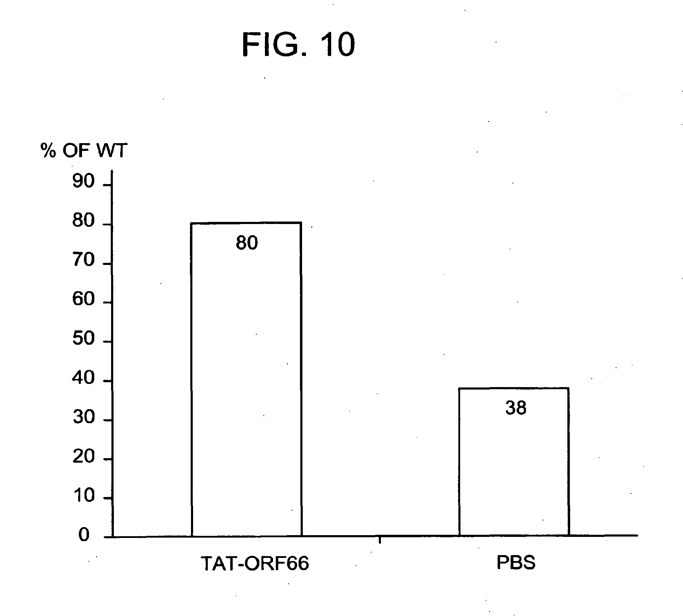

**Figure 10****:** Complex I activity is restored in cells from patients with complex I deficiency that are treated with TAT-ORF66. "PBS" refers to untreated cells.

### DETAILED DESCRIPTION OF THE INVENTION

In one embodiment, the present invention provides a fusion protein for use in a method of treatment as defined in the claims. In certain preferred embodiments, the fusion protein is produced by recombinant techniques As provided herein, provision of PTD-fusion proteins containing a catalytic domain of a mitochondrial enzyme to a subject in need thereof is capable of treating and alleviating mitochondrial metabolic disorders.

The fusion protein comprises a mitochondria targeting sequence (MTS). The MTS is an MTS of another mitochondrial enzyme that is encoded by the nuclear DNA, translated/produced in the cytoplasm, and transported into the mitochondria. It will be understood to those skilled in the art that the MTS's of various mitochondrial enzymes synthesized in from nuclear genes are largely if not completely interchangeable, and thus may be used in an interchangeable fashion in methods and compositions of the present invention.

The MTS is situated between the PTD and the functional component as the case may be. In certain more preferred embodiments, the portion of the fusion protein C-terminal to the MTS consists of the functional component of an enzyme. In another embodiment, no residues heterologous to the enzyme are present C-terminal to the MTS. In this embodiment, cleavage of the MTS generates an enzyme with the native sequence, thus able to readily integrate into a conformationally-sensitive multi-component enzyme complex.

The PTD is a TAT peptide. Non-limiting representative examples of suitable PTD sequences are listed herein.

Each type of fusion protein for use in the method of treatment represents a separate embodiment of the present invention.

Described herein is a pharmaceutical composition for treating or alleviating a mitochondrial disorder, comprising a pharmaceutically acceptable carrier and as an active ingredient the fusion protein.

Also described herein is the use of a fusion protein of the present invention for the preparation of a medicament for the treatment of a mitochondrial disorder.

Also described herein is a method for treating a mitochondrial disorder, the method comprising the step of administering to a subject in need of such treatment a therapeutically effective amount of a fusion protein of the present invention, thereby treating a mitochondria disorder. Upon entry into a mitochondrion of the subject, the fusion protein restores the missing enzymatic activity.

Also described herein is a method for introducing a mitochondrial enzyme activity into a mitochondria of a subject, the method comprising the step of administering to a subject in need of such treatment a therapeutically effective amount of a fusion protein of the present invention, thereby introducing a mitochondrial enzyme activity into a mitochondria of a subject in need thereof.

As provided herein in Examples 1-4, TAT-LAD is able to enter cells and their mitochondria rapidly and efficiently. Moreover, it is able to raise LAD activity within LAD-deficient cells and their mitochondria back to normal activity values and higher. Most importantly, it is able to replace the mutated enzyme and be naturally incorporated into α-ketoacid dehydrogenase complexes such as the PDHC. We show here that PDHC activity of LAD deficient cells treated with TAT-LAD changed from ∼10% to 70-75% of normal activity after only 3Hr' of incubation. These high enzymatic activity values decreased following 24Hr' of incubation but stably remained well above basal activity. Thus, in a clinical context, a single application may be sufficient for a patient presenting with a life-threatening decompensation episode.

One advantage of using TAT-fusion proteins for treatment of mitochondrial disorders is their ability to be delivered into virtually all cells with no specificity. When trying to replace a mutated mitochondrial enzyme there is no need for specific targeting but rather to deliver the enzyme into each cell/tissue, reaching primarily high-energy demanding tissues such as muscles, liver, and central nervous system (CNS), which are usually the most affected in these types of disorders.

Moreover, LAD-TAT exhibited a very rapid mode of action, raising whole-cell LAD activity in LAD-deficient cells back to normal values after only 30 min incubation and even higher values upon prolongation of treatment (Figure 2D-E). Normal LAD activity in fibroblasts ranges between 60-140nmol/min/mg and in asymptomatic carriers of LAD deficiency between 25-50nmo/min/mg (Berger, 1996).

The PDHC is a macromolecular multi-component enzymatic machine. Its assembly process involves numerous different subunits. Optimal positioning of individual components within this multi-subunit complex directly affects the efficiency of the overall enzymatic reaction and the stability of its intermediates (Vettakkorumakankav, 1996; Berger, 1996; Del Gaizo 2003b). Given the structure of the complex, restoration of activity of a whole complex reduced due to a single mutated nonfunctioning component would not have been expected to be treatable by exogenous administration of the mutated component. Interestingly, as demonstrated herein, TAT-mediated replacement of the E3 component was sufficient to increase the enzymatic activity of the whole complex of the PDHC (Figure 5).

As provided herein, PTD fusion proteins for use of the present invention raised PDHC activity four- to fivefold in a sustained fashion, through the last timepoint at 24 hours (Figure 5B). When treating a metabolic disease such as LAD deficiency, there is no need to augment enzyme activity back to 100%; rather, it need by raised above the energetic threshold required for a normal metabolism. Even slight augmentation in LAD activity can raise ATP synthesis rate and can favorably affect the neurological involvement in LAD deficiency. Therefore the changes demonstrated herein in LAD activity, LAD/CS ratio, and PDHC activity are likely to significantly affect clinical presentation in patients at least to the level of asymptomatic LAD deficiency carriers.

Today, one major impediment of ERT is the inability of the administered enzyme to cross the blood-brain barrier (BBB). This fundamental obstacle has severely limited development of ERT for metabolic disorders in which the CNS is affected (Brady, 2004). TAT-fusion proteins are able to cross BBB, thus making them a favorable choice for development of ERT for metabolic disorders involving the CNS.

As provided herein in Examples 5-7, the LAD deficiency of E3 mice is treatable by PTD-LAD proteins for use of the present invention. It is noteworthy that experiments with the E3 mice have established substantial evidence that alternations in α-ketoacid dehydrogenases (the complexes containing LAD) may play a role in the pathogenesis of neurodegenerative diseases. Decreases in activity of the LAD-associated complexes α-ketoglutarate dehydrogenase and pyruvate dehydrogenase, in brain, represent a common element in several age-associated neurodegenerative diseases, including Alzheimer's and Parkinson's diseases (Gibson et al., 2000 and Sullivan and Brown, 2005). Studies of adult LAD-deficient mice have suggested that a partial decrease of LAD, which is sufficient to diminish activity of its associated enzyme complexes (Johnson et al., 1997), results in an elevated level of susceptibility to chemical neurotoxicity (Klivenyi et al., 2004). Moreover, variations in the *DLD* gene (the mouse analogue of LAD) have been linked to Alzheimer's disease (Brown et al, 2004 and Brown et al, 2007). Furthermore, PTD-LAD fusion proteins of the present invention are shown herein to restore LAD and PDHC activity to brain, thus showing that they can cross the BBB and functionally integrate into PDHC there. These results clearly show that PTD-LAD fusion proteins of the present invention are capable of treating neurodegenerative diseases.

### EXPERIMENTAL DETAILS SECTION

### MATERIALS AND EXPERIMENTAL METHODS (Examples 1-4)

### Cell culture

Fibroblast primary culture cells of patients bearing the mutated genotypes G229C/Y35X, E375K/E357K and D479V/D479V were established from forearm skin biopsies. Cells were maintained in DMEM (Biological Industries, Beit-Haemek, Israel) supplemented with 15% Fetal Bovine Serum (HyClone, Logan UT, USA), penicillin/streptomycin and L-glutamine (Biological Industries, Beit-Haemek, Israel) in a humified atmosphere with 5% CO₂ at 37°C. All cell cultures tested negative for mycoplasma contamination. All experiments involving patients' cells were approved by the Hadassah University Hospital ethical review committee.

### Construction of plasmids expressing TAT-LAD and LAD proteins.

TAT fusion proteins were generated using the pTAT plasmid, provided by Dr. S.F. Dowdy. The plasmid contains a gene encoding a 6-histidine His-tag, followed by the TAT peptide (AA 47-57). To construct a pTAT plasmid with LAD fused to the His-tagged TAT peptide, the gene for human LAD precursor was amplified by PCR from a placental cDNA library using the oligonucleotides set forth in SEQ ID NO: 1 (forward) and SEQ ID NO: 2 (reverse). The PCR product was cloned downstream of the TAT sequence into a *BamHI*/*XhoI*-digested pTAT vector.

The TAT-Δ-LAD expression plasmid was constructed by PCR amplification of the mature LAD sequence from the TAT-LAD plasmid using the oligonucleotides set forth in SEQ ID NO: 5 (forward) and SEQ ID NO: 6 (reverse). The PCR product was cloned downstream of the TAT sequence into a BamHI/XhoIcut pTAT plasmid.

A control LAD protein lacking the TAT peptide was also cloned. The LAD expression vector was generated by subcloning the LAD fragment into a modified pTAT vector lacking the TAT sequence; nucleotide and amino acid sequences of the control LAD protein are set forth in (SEQ ID NO: 45-46, respectively). All clones were confirmed by sequencing analysis. Examples of the sequences used are given below:

The TAT-LAD DNA sequence- (includes His tag, TAT peptide, and the gene for human LAD precursor) is set forth in SEQ ID NO: 3. The amino acid sequence is set forth in SEQ ID NO: 4.

The naturally-occurring LAD MTS has the sequence set forth in SEQ ID NO: 39. The sequence used in TAT-LAD is identical except that it lacks the N-terminal Met and is set forth in SEQ ID NO: 41.

### Expression and purircation of Proteins

*E. coli* BL21-CodonPlus (λDE3) competent cells transformed with plasmids encoding the fusion proteins were grown at 37°C in SLB medium containing kanamycin (50µg/ml), tetracycline (12.5µg/ml) and chloramphenicol (34µg/ml). At an OD₆₀₀ of 0.8, protein expression was induced by adding IPTG (1mM, final concentration). After a 24-hr incubation at 22°C, cells were harvested by centrifugation (2000Xg for 15 min at 4°C) followed by sonication in binding buffer (PBS pH7.4, PMSF 1mM and 10mM imidazole (Sigma-Aldrich, St. Louis, USA)). The suspensions were clarified by centrifugation (35,000Xg for 30 min at 4°C), and the supernatants containing the fusion proteins were purified under native conditions using HiTrap™ Chelating HP columns (Amersham-Pharmacia Biotech, Uppsala, Sweden) pre-equilibrated with binding buffer. Columns were washed by stepwise addition of increasing imidazole concentrations. Finally, target proteins were eluted with elution buffer (PBS pH7.4 and 500mM Imidazole). All purification procedures were carried out using the ÄKTA™ FPLC system (Amersham-Pharmacia Biotech, Uppsala, Sweden). Removal of imidazole was performed by dialysis against PBS (pH 7.4). Proteins were kept frozen in aliquots at -20°C until use.

### Western blot analysis

Proteins (5-20µg protein/lane) were resolved on 12% SDS-PAGE gels and transferred onto an Immobilon-P™ Transfer membrane (Millipore, Bradford, USA). Western blots were performed using anti-LAD (Elpeleg 1997), anti-His (Amersham-Pharmacia Biotech, Uppsala, Sweden), anti-α-Tubulin (Serotec, Oxford, UK) and anti-VDAC (porin) (Calbiochem, Darmstadt, Germany) antibodies at 1:1000, 1:10,000, 1:10,000, or 1:5000 dilutions, respectively.

### Delivery of fusion proteins into cells

Cells were plated on 6-well plates or in 250ml flasks (NUNC Brand Products, Roskilde, Denmark). When cells reached 90% confluency, medium was replaced with fresh medium containing 0.05-0.1mg/ml (final concentration) TAT-fusion proteins for various time periods. After incubation, cells were washed with PBS, trypsinized, pelleted and kept at -80°C till further use. Pellets were then resuspended in PBS containing 0.5% Triton X-100 and 1mM PMSF (Sigma-Aldrich, St. Louis, USA), kept on ice for 10 minutes and centrifuged at 15,000Xg for 10 minutes. The supernatants were analyzed by western blotting analysis or for enzyme activity.

### Isolation of sub-cellular fractions

Mitochondrial fractions were isolated from cultured cells using a differential centrifugation technique (Bourgeron 1992). Cells were washed with PBS, tripsinized and pelleted. The cells' pellets were kept frozen at -80°C till use. Pellets were resuspended in ice-cold Tris-HCl buffer (10mM, pH7.6, 1mM PMSF) and homogenized with a Dounce homogenizer (Teflon-glass). The homogenates were combined with sucrose (0.25M, final concentration) and centrifuged for 10 min at 600 X g at 4°C. The supernatants were collected and centrifuged for 10 min at 14,000 X g at 4°C. The resulting pellets containing the mitochondria were resuspended in PBS containing 0.5% Triton X-100 and 1mM PMSF and incubated on ice for 15 min before being analyzed for enzymatic activities and Western blots. Purity of sub-cellular fractions was confirmed by Western blotting using the following specific marker antibodies: α-tubulin for cytoplasm and VDAC (porin) for mitochondria.

### LAD and Citrate Synthase (CS) activity assays

LAD and CS activities were determined for whole-cell protein extracts, sub-cellular fractions or purified TAT-fusion proteins.

LAD activity was determined as described in Berger, 2005. The reaction was performed in potassium phosphate buffer (50mmol/l, pH 6.5) containing EDTA (Immol/l) and NADH (1.5mmol/l) (Sigma-Aldrich, St. Louis, USA). Following addition of Lipoamide (2mmol/l) (Sigma-Aldrich, St. Louis, USA), the decrease in absorbance from a steady state was measured spectrophotometrically at 340nm (Uvikon XL, Bio-Tek Instruments, Milan, Italy).

CS activity was determined by following spectrophotometrically (412nm) the appearance of free SH-group of the released CoA-SH upon the addition of 10mM oxaloacetate to sub-cellular fractions to which 100uM acetyl-CoA and 2mM DTNB (Dithionitrobenzoic acid; Sigma-Aldrich, St. Louis, USA) was added.

### Analysis of cells treated with TAT-LAD by fluorescence and confocal microscopy

TAT-LAD and LAD proteins were fluorescently labeled with Fluorescin (FITC) using a protein labeling kit (EZ-Label, PIERCE Biotechnology, Rockford IL, USA) according to the manufacturer's protocol. Unbound fluorescent dye was removed by dialysis against PBS. Cells grown on coverslips to 50-70% confluency were treated with FITC-labeled TAT-LAD or LAD (0.1mg/ml, final concentration) for various time periods. When indicated, cells were further incubated with the mitochondrial selective fluorescent dye MitoTracker-Red CMXRos™ (Molecular Probes, Eugene, USA, 200nM). Cells were then washed with PBS, fixed in 3.7% formaldehyde in PBS for 10min at room temperature, and washed again. In fluorescence experiments, cells were analyzed directly without fixation. Cells were analyzed with a fluorescence microscope (NIKON 90i, Nikon Corporation, Tokyo, Japan) or a confocal laser scanning microscope (NIKON C1, Nikon Corporation, Tokyo, Japan).

### PDHC activity assay

PDHC activity was determined using radioactive pyruvate as follows: Frozen cell pellets were suspended and sonicated in 0.25ml potassium-phosphate buffer (10mM, pH 7,4). The reaction was performed in 0.4ml reaction buffer containing 200-300µg protein whole-cell extracts and was terminated by adding 1M perchloric acid. The ¹⁴CO₂ was collected in Hyamine Hydroxide™ (Packard, USA) and counted in a liquid scintillation (UltimaGold™, Packard, USA) counter (Kontron Instruments, Zurich, Switzerland). Controls with no coenzymes were conducted simultaneously to account for background ¹⁴CO₂ release.

### Delivery and processing of the fusion proteins

Mitochondria isolated from healthy fibroblasts and radioactive-labeled TAT-LAD protein and control TAT-Δ-LAD protein were used. *In vitro* translation of the proteins was performed using the TnT Quick Coupled Transcription/Translation System™ (Promega, Madison, WI) in the presence of [³⁵S]-methionine (Amersham Biosciences, Piscataway, NJ). Isolated mitochondria were incubated with the radio-labeled proteins (1 mg/ml mitochondria, 1:10 volume-to-volume ratio) for 30 minutes at 30°C, then pelleted, washed with buffer A, and treated with 2.5 µg/ml proteinase K (Roche Diagnostics, Mannheim, Germany) for 10 minutes on ice. Phenylmethylsulphonylfluoride was added (1 mmol/l, final concentration) to stop the reaction. Mitochondria were then re-pelleted, washed, and analyzed using 12% sodium dodecyl sulfate polyacrylamide gel electrophoresis gels that were fixed, dried and visualized using a PhosphorImager™ (BAS-2500; FujiFilm, Valhalla, NY).

### EXAMPLE 1: Construction, expression, purification and in-vitro activity of TAT-LAD and LAD proteins

Over-expression and purification of the fusion protein TAT-LAD was accomplished by inserting the precursor human LAD sequence into the pTAT vector. Expression vectors encoding TAT-Δ-LAD, lacking the MTS sequence, and a control LAD protein lacking the TAT peptide were also constructed (Figure 1A). These proteins were all expressed and highly purified under the same conditions. Sodium dodecyl sulfate polyacrylamide gel electrophoresis analysis and Western blotting confirmed the identity of these highly purified proteins (Figure 1B). These purified LAD-based fusion proteins were found to be highly active in an *in vitro* LAD enzymatic activity assay (Figure 1C).

### EXAMPLE 2: Delivery of TAT-LAD into LAD deficient cells

The next experiment examined the ability of protein transduction domains (PTD's) such as TAT to deliver the human LAD enzyme into cultured cells from patients with LAD deficiency. Purified TAT-LAD was incubated for different time periods with cells from patients heterozygous for the G229C/Y35X and E375K LAD mutations. Whole-cell protein extracts were prepared and analyzed by Western blotting using anti-LAD antibodies. TAT-LAD fusion protein (58kDa) rapidly entered G229C/Y35X cells and was detectable after 30 minutes of incubation (Figure 2A). In cells homozygous for the E375K mutation (Figure 2B), its delivery was somewhat slower; it was detected within the cells after a 2-hour incubation. Endogenous mutated LAD (50kDa) was detected only in G229C/Y35X cells and not in E375K cells (Figure 2A). In both cell lines, steady state was reached after 2-3 hours; thus, the amount of the fusion protein remained similar through the 6-hour (Figure 2A) and 24-hour (Figure 2B) timepoints.

Delivery of TAT-LAD into cells was also followed using direct fluorescence analysis. TAT-LAD was fluorescently labeled with Fluorescin (FITC), incubated with G229C/Y35X cells for different time periods, and analyzed by fluorescence microscopy. FITC-labeled LAD protein lacking the PTD moiety was used as a control protein. TAT-LAD was efficiently delivered into the cells (Figure 2C, panels 1-3) whereas fluorescence signals were not detected in cells treated with the control LAD protein (Figure 2C, panel 4). These results correlated with the Western blot analysis (Figure 2A-B). TAT-LAD was detected rapidly within cells (after only 30 min of incubation; Figure 2C, panel 1) and there were no differences in fluorescence signal intensity after longer incubation periods (Figure 2C, panels 2-3).

To test the ability of a PTD to deliver an active human LAD enzyme into LAD-deficient cells, purified TAT-LAD was incubated with G229C/Y35X and E375K cells for different time periods. These experiments utilized the control LAD protein and TAT-PAH protein, which is a control TAT-fusion enzyme that lacks LAD activity. Protein extracts of treated cells were analyzed for their LAD activity. Activity of LAD within the cells increased dramatically in concordance with incubation time, reaching steady state after 2-3Hr' (Figure 2D-E). These results resembled those observed by the Western analysis. This augmentation in LAD activity within patients' cells was dose-dependent, and was not observed following addition of control LAD protein.

In G229C/Y35X cells, LAD activity increased by 2.5-fold (from 31nmol/min/mg to 78nmol/min/mg) after only 30 min of incubation and reached equilibrium of 230-250nmol/min/mg, an 8-fold increase, after 2-3Hr' (Figure 2D). G229C/Y35X cells incubated with control proteins TAT-PAH or LAD showed no change in basal LAD activity, >20nmol/min/mg, which is lower than normal values (Saada 2000). In E375K cells (Figure 2E), the same trends were observed. LAD activity increased by -9 fold (increasing from 5nmol/min/mg to 423nmol/min/mg) after 2Hr' of incubation and reached equilibrium of 630-690nmol/min/mg after 4Hr' of incubation, which lasted through the last time 24Hr' incubation. E375K cells that were incubated with the control protein LAD showed no change in their basal LAD activity.

Though treated with identical protein concentrations, E375K and G229C/Y35X cells responded differently as maximum activity values were much higher in E375K than in G229C/Y35X cells, indicating possible differences in treatment efficiency in patients bearing different genotypes.

### EXAMPLE 3: Delivery of TAT-LAD into mitochondria

The next step was to examine the ability of TAT-LAD to be delivered across the mitochondrial membrane and naturally processed in mitochondria. *In vitro*-translated [³⁵S]-methionine- labeled TAT-LAD was incubated with isolated mitochondria from healthy fibroblasts. The mitochondria were treated with proteinase K to digest proteins nonspecifically adsorbed to the outer membrane, thereby ensuring that the mitochondrial extract contained only proteins within the mitochondria. As a control, ³⁵S-methionine-labeled TAT-Δ-LAD protein lacking the MTS (and consequently lacking the natural processing site within it) was used. As seen in Figure 3A, TAT-LAD and TAT-Δ-LAD were both expressed at their expected molecular sizes of 58 and 54 kd, respectively. After treatment, both TAT-LAD and TAT-Δ-LAD were detected within the mitochondria after 30 minutes of incubation (**Figure 3B**), because of the PTD sequence that these proteins carry. However, only the TAT-LAD fusion protein was processed to its mature size, as indicated by the appearance of an additional 50-kd band on the SDS-PAGE autoradiograph (**Figure 3B**, asterisk). As expected, the TAT-Δ-LAD protein (lacking the MTS) was not processed, and appeared as a single band at its full unprocessed size. Thus, TAT-LAD is able to be delivered into mitochondria and processed therein.

It was next examined whether TAT-LAD was able to reach mitochondria after being delivered into intact cells. Purified TAT-LAD was incubated with G229C/Y35X and D479V cells for different time periods. After incubation, mitochondrial and cytoplasmic sub-cellular fractions were prepared and analyzed for presence of TAT-LAD and for LAD enzymatic activity. CS activity was utilized as a mitochondrial marker. Western blot of sub-cellular fractions indicated the presence of TAT-LAD (58kDa) in both cytosolic and mitochondrial fractions of treated G229C/Y35X and D479V cells following 4 and 6Hr' of incubation (Figures 4D and H, respectively). Purity of sub-cellular fractions was confirmed using antibodies against the sub-cellular markers α-tubulin (50kDa) for the cytoplasm and VDAC (porin) (31kDa) for the mitochondria.

In support of these findings, there was a significant increase in LAD activity in both cytosolic and mitochondrial fractions of cells treated with TAT-LAD.

In G229C/Y35X cells, LAD activity in mitochondrial fractions increased by 7-fold (from 28nmol/min/mg to 205nmol/min/mg) after a 4Hr' incubation (Figure 4A). Enzymatic activity remained about the same after 6Hr' (193nmol/min/mg) demonstrating that equilibrium had been reached. This dramatic increase in LAD activity was also measured in cytosolic fractions, changing from 10nmol/min/mg to 222 and 339 nmol/min/mg after 4Hr'-and 6Hr' incubations, respectively (Figure 4A). Similar results were observed with D479V cells. LAD activity in mitochondrial fractions changed from 28nmol/min/mg to 165 and 117 nmol/min/mg after 4Hr'-and 6Hr' incubations, respectively (Figure 4E). In cytosolic fractions, activity changed from 20nmol/min/mg to 125 and 193 nmol/min/mg after 4Hr'-and 6Hr' incubations, respectively.

In addition, CS enzymatic activity was determined in G229C/Y35X cells (Figure 4B) and D479V cells (Figure 4F). CS is a mitochondrial matrix enzyme that participates in the Krebs cycle, converting Acetyl-CoA to Citrate. CS enzymatic activity assay was used as a control reference to verify the purity of mitochondrial sub-fractions and also to calculate LAD/CS ratio to standardize LAD enzymatic activity values. In both cell lines, CS activity in cytosolic fractions was barely detectable, while in the mitochondrial fractions it was within the range of normal levels for fibroblasts, thus verifying the purity of sub-cellular fractions. Furthermore, CS activity was constant and almost identical in all mitochondrial fractions, enabling proper standardization of LAD activity values. Mitochondria of G225C/Y35X exhibited LAD/CS ratios of 0.102 before incubation and 0.740 and 0.678 after 4 Hr'-and 6 Hr' incubations with TAT-LAD, respectively (Figure 4C). In mitochondria of D479V treated cells, the LAD/CS ratio changed from 0.142 to 0.715 and 0.561 after 4Hr'-and 6Hr'incubation, respectively (Figure 3G).

Co-localization experiments were used to further confirm delivery of TAT-LAD into the mitochondria of LAD-deficient cells. FITC-labeled TAT-LAD was incubated with G229C/Y35X cells grown on coverslips for different time periods. Cells were then incubated with the mitochondrial-selective fluorescent dye MitoTracker-Red CMXRos™ and analyzed by confocal microscopy. As shown in Figure 5A, TAT-LAD (green fluorescence, middle column) co-localized with mitochondria (red fluorescence, left column) within the first 30 minutes of incubation, as indicated by the yellow staining in the merge (right column).

### EXAMPLE 4: A PTD-LAD fusion protein augments PDHC activity in LAD-deficient cells

The final and most crucial test for TAT-LAD's ability to successfully treat LAD deficiency by ERT is the enzyme's ability to substitute for the mutated endogenous enzyme, including successful integration into its natural multi-component enzymatic complexes such as pyruvate dehydrogenase complex (PDHC). LAD deficiency affects three mitochondrial multi-component enzymatic complexes, whose activity could be restored by TAT-LAD. The ability of TAT-LAD to successfully replace the endogenous defective enzyme and increase the activity of PDHC was tested in D479V and E375K cells.

PDHC activity was increased in the two genotypically different cells. In E375K cells, PDHC activity increased significantly by 12-fold after 3 hours of incubation (from 0.029 to 0.367 nmol/min/mg), remaining approximately four- to fivefold higher than the low basal values for at least 24 hours (Figure 5B). Presented as a percentage of normal PDHC activity of healthy fibroblasts, the PDHC activity in D479V cells increased from 9% to 69% of normal activity after 3 hours of incubation, remaining at 50% of the normal level for at least 24 hours. In E375K cells, PDHC activity increased from 5 to 75% of normal activity after 3 hours incubation, declining to about 30% after 24 hours of incubation (Figure 5C). Of note, these PDHC activity values are in close correlation with LAD enzymatic activity values measured in mitochondria of treated cells, reaching maximum levels after 3Hr' incubation with TAT-LAD.

PTD-LAD fusion proteins are thus able to treat LAD deficiency by augmenting PDHC activity in LAD-deficient cells.

### EXAMPLE 5: Enzymatic Activity of LAD in plasma of E3 mice injected with TAT-LAD

### MATERIALS AND EXPERIMENTAL METHODS (Examples 5-6)

The mouse model of LAD deficiency is described in Klivenyi, P. et al (Mice deficient in dihydrolipoamide dehydrogenase show increased vulnerability to MPTP, malonate and 3-nitropropionic acid neurotoxicity. J Neurochem 88: 1352-1360, 2004) and Johnson, MT et al (Targeted disruption of the murine dihydrolipoamide dehydrogenase gene (Dld) results in perigastrulation lethality. Proc Natl Acad Sci USA 94: 14512-14517, 1997). These mice are heterozygotes to a recessive loss-of-function mutation affecting LAD gene (*Dld*, in mice) expression at the mRNA level (instability) (*Dld+*/*-* mice or E3 mice). Homozygous mice die *in-utero* at a very early gastrulation stage. These mice are phenotypically normal, though their LAD activity is reduced by ∼50%, affecting all the LAD-dependent enzyme complexes. Similarly, humans heterozygous for LAD deficiency exhibit ∼50% LAD activity, but usually have no clinical symptoms. These mice are currently used in experiments in the field of neurodegenerative disorders including Alzheimer's, Parkinson's and Huntington's disease.

A single dose (0.2mg per mouse) of highly purified TAT-LAD was injected into the tail vein of E3 mice, and several tissues were extracted and analyzed for LAD and PDHC activities at different time points. Several mice were used at each time point.

### RESULTS

To test the ability of TAT-LAD to treat LAD deficiency *in vivo*, purified TAT-LAD was injected intravenously into E3 mice, and its effect on LAD and PDHC activities was measured in several tissues. This experiment concentrated our on 3 major organs that have the highest energy demands and thus are often affected in mitochondrial disorders - the liver, the heart (muscles) and the brain.

First, behavior and stability of the injected fusion protein TAT-LAD in the plasma of injected mice were characterized by measuring LAD enzymatic activity. Blood samples from E3 injected mice were withdrawn at different time points, and plasma was prepared.

No LAD activity is present in the plasma of either normal healthy mice or E3 mice, so the LAD activity at the first time point was set as the reference. Following the first time point, a decrease in LAD activity was observed in the plasma of E3 mice, over time (Figure 6). To determine whether a component or factor exists in the plasma that reduced LAD activity over time, mouse plasma was incubated with TAT-LAD *in vitro* under the same concentrations: at 37°C and for the same time periods. LAD activity remained stable in these plasma samples. Thus, the decrease in LAD enzymatic activity in the plasma was a result of delivery of TAT-LAD into the organs and tissues of mice. Indeed, these results correlate with the LAD activity measured within the organs (Figure 7 below). The LAD control protein, lacking the TAT delivery moiety, also decreased its activity in plasma overtime, suggesting possible clearance mechanisms in this case.

### EXAMPLE 6: TAT-LAD increases LAD activity in organs of LAD-deficient mice

Organs were harvested from the mice described in the previous Example, and LAD activity there was measured. Figure 7A depicts the percentage increase from the basal activity measured in the heterozygous mice, namely E3, non-treated mice, injected only with PBS. A single intravenous injection of TAT-LAD (0.2mg per mouse) significantly increased LAD enzymatic activity within the liver, heart and most importantly - in the brain after only 30 minutes. The shapes of the curves were similar in the brain and heart and slightly different in the liver (Figure 7C-D and B, respectively).

Even more robust increases were observed at steady state. In liver, LAD activity reached a steady state at about 40% of non-treated mice and remained at the same level for up to 6 hours, while in brain and heart, steady-state LAD activity was higher, peaking at 4 hours at levels of 80% and 100%, respectively. The LAD control protein, lacking the TAT delivery moiety, injected in the same amount and under identical conditions, did not significantly increase in LAD activity in the organs. In addition and also of importance was the fact that 24 hours following the injection, LAD activity was still 10% higher than the basal activity.

Thus, PTD-LAD fusion proteins are able to fully restore deficient LAD activity in a LAD-deficient disease model and thus are able to treat acute decompensation episodes. The long-term magnitude of the increase after only a single treatment, 10%, is also sufficient to affect the clinical status of many cases.

### EXAMPLE 7: TAT-LAD increases PDHC activity in organs of LAD-deficient mice MATERIALS AND EXPERIMENTAL METHODS

Principle of PDHC activity measurement in mice's tissues. A kit from Mitosciences™ (Catalog No. MSP18) for measuring PDHC enzymatic activity was used. PDHC was immuno-captured from tissue lysates, and its enzymatic activity is measured. This ensured that any increase in the measured PDHC activity resulted only from the TAT-LAD that had become integrated into the PDHC complex. The enzymatic assay measures reduction in NAD⁺ to NADH by an increase in absorbance at 340nm.

### RESULTS

The next experiment directly tested the ability of PTD-LAD fusion proteins to substitute for the mutated endogenous enzyme, following successful integration into its natural multi-component enzymatic complexes, in the organs of the TAT-LAD-injected mice described in Example 5. Figure 8A depicts the percentage increase over basal PDHC activity of untreated E3 mice (mock-treated by injection with PBS) in each organ. Brains and hearts (Figure 8C-D, respectively) of treated E3 mice both responded robustly to TAT-LAD treatment; peaking at 4 hours, with a 145% increase in PDHC enzymatic activity; liver samples (Figure 8B) peaked at 2 hours with a 135% increase in the activity. A substantial and significant increase in PDHC enzymatic activity (40-65%) was also evident in the three organs at 24 hours posttreatment. Treatment with the control protein LAD did not affect the basal PDHC activity. Interestingly, the percent increase in PDHC activity was much greater than that of LAD activity in the tissues, highlighting the potency of the fusion proteins used (Figure 9A-C).

Thus, a single application of a PTD-LAD fusion protein is able to significantly increase PDHC activity in a disease model of LAD deficiency. PTD-LAD fusion proteins are thus able to treat and ameliorate LAD deficiency pathologies.

### EXAMPLE 8: TAT-ORF66 restores Complex I activity in the cells of a patient with NADH:ubiquinone oxidoreductase (Complex I) deficiency

### MATERIALS AND EXPERIMENTAL METHODS

In order to construct a plasmid expressing a TAT-C6ORF66 fusion, the gene for human C6ORF66 was amplified by PCR from lymphocytes complementary DNA library, using the oligonucleotides set forth in SEQ IN NO: 47 (forward) and SEQ IN NO: 48 (reverse). The PCR product was cloned downstream of the TAT sequence into a BamHI/XhoI-digested pTAT fragment.

### RESULTS

A missense mutation in a conserved residue of the C6ORF66 gene has been identified in a consanguineous family that presented with infantile mitochondrial encephalomyopathy attributed to isolated NADH:ubiquinone oxidoreductase (Complex I) deficiency. In muscle of patients, levels of the C6ORF66 protein and of fully assembled Complex I were markedly reduced. Transfection of the patients' fibroblasts with wild-type C6ORF66 cDNA restored complex I activity (Saada A et al, C6ORF66 is an assembly factor of mitochondrial complex I. Am J Hum Genet 82(1):32-8, 2008).

The mRNA sequence of C6ORF66 is set forth in SEQ ID NO: 7 (GenBank Accession # NM_014165).

The amino acid sequence of the product of C6ORF66 is set forth in SEQ ID NO: 8 (GenBank Accession # NM_014165). The first 34 residues of the protein, (SEQ ID NO: 9), are predicted by the TargetP software to form the mitochondrial-targeting sequence (Saada A et al, ibid).

To test the ability of a TAT-fusion protein to treat Complex I deficiency, a TAT-C6ORF66 fusion protein was constructed and highly purified. Primary fibroblast cells isolated from a patient with the missense mutation in the C6ORF66 gene were incubated with TAT-ORF66 for 48 hr, and mitochondria were isolated and analyzed for complex I activity. The TAT-fusion protein was able to restore 80% of wild-type complex I activity in the mitochondria (Figure 10).

Thus, Complex I deficiency is treatable using TAT-fusion proteins.

The findings presented herein demonstrate that a variety of mitochondrial enzymes can be successfully treated by ERT using PTD-based fusion proteins. Deficiencies in LAD, an enzyme that forms part of several multi-component enzymatic complexes, and C6ORF66, an assembly factor of Complex I, were successfully treated. Of note, the enzymes were able to translocate into the mitochondria and function in the conformation-sensitive context of these enzymatic complexes with their activity intact, following removal of the heterologous parts of the molecule.

The findings presented herein demonstrate that a variety of mitochondrial metabolic disorders are treatable by ERT using PTD-based fusion proteins, as evidenced by treatment of both LAD deficiency and Complex I deficiency.

### References

Chinnery, P.F. & Schon, E.A. Mitochondria. J. Neurol. Neurosurg. Psychiatry 74, 1188-1199 (2003).
Brautigam, C.A., Chuang, J.L., Tomchick, D.R., Machius, M. & Chuang, D.T. Crystal structure of human dihydrolipoamide dehydrogenase: NAD+/NADH binding and the structural basis of disease-causing mutations. J. Mol. Biol. 350, 543-552 (2005).
Vettakkorumakankav, N.N. & Patel, M.S. Dihydrolipoamide dehydrogenase: structural and mechanistic aspects. Indian J. Biochem. Biophys. 33, 168-176 (1996).
Berger, I., Elpeleg, O.N. & Saada, A. Lipoamide dehydrogenase activity in lymphocytes. Clin. Chim. Acta. 256, 197-201 (1996).
Elpeleg, O.N. et al. Lipoamide dehydrogenase deficiency: a new cause for recurrent myoglobinuria. Muscle Nerve 20, 238-240 (1997).
Shaag, A. et al. Molecular basis of lipoamide dehydrogenase deficiency in Ashkenazi Jews. Am. J. Med Genet. 82, 177-182 (1999).
Saada, A., Aptowitzer, I., Link, G. & Elpeleg, O.N. ATP synthesis in lipoamide dehydrogenase deficiency. Biochem. Biophys. Res. Commun. 269, 382-386 (2000).
Brady, RO. & Schiffmann, R. Enzyme-replacement therapy for metabolic storage disorders. Lancet Neurol. 3, 752-756 (2004).
Futaki, S. et al. Arginine-rich peptides. An abundant source of membrane-permeable peptides having potential as carriers for intracellular protein delivery. J. Biol. Chem. 276, 5836-5840 (2001).
Kuppuswamy M et al, Multiple functional domains of Tat, the trans-activator of HIV-1, defined by mutational analysis. Nucleic Acids Res 17(9):3551-61, 1989).
Del Gaizo, V., MacKenzie, J.A. & Payne, R.M. Targeting proteins to mitochondria using TAT. Mol. Genet. Metab. 80, 170-180 (2003a).
Del Gaizo, V. & Payne, R.M. A novel TAT-mitochondrial signal sequence fusion protein is processed, stays in mitochondria, and crosses the placenta. Mol. Ther. 7, 720-730 (2003b).
Bourgeron, T., Chretien, D., Rotig, A., Munnich, A. & Rustin, P. Isolation and characterization of mitochondria from human B lymphoblastoid cell lines. Biochem. Biophys. Res. Commun. 186, 16-23 (1992).

## Claims

1. A fusion protein for use in a method of treatment of a mitochondrial disorder in a subject,
said fusion protein comprising a protein transduction domain (PTD) fused to a functional component of a mitochondrial enzyme and a mitochondria targeting sequence (MTS),
wherein said MTS is situated between said PTD and said functional component and
wherein said MTS is an MTS of another mitochondrial enzyme that is encoded by a nuclear gene,
wherein said mitochondrial enzyme is an enzyme of a mitochondrial multi-component enzyme complex,
and
wherein said protein transduction domain is a TAT peptide,
wherein said treatment comprises administering said fusion protein to said subject and is a continuous prolonged treatment for a chronic disease or comprises a single or few time administrations for treatment of an acute condition.

2. The fusion protein for use according to claim 1, wherein the portion of said fusion protein that is C-terminal to said MTS consists of said functional component of an enzyme.

3. The fusion protein for use according to claim 2 wherein there are no residues heterologous to the enzyme present C-terminal to the MTS and cleavage of the said MTS generates an enzyme with the native sequence, which is readily integrated into a conformationally-sensitive multi-component enzyme complex.

4. The fusion protein for use of claim 1 or 2, wherein said enzyme is Lipoamide Dehydrogenase (LAD).

5. The fusion protein for use of claim 1 or 2, wherein said enzyme is selected from the group consisting of 2-oxoisovalerate dehydrogenase alpha subunit (Branched-Chain Keto Acid Dehydrogenase E1α), 2-oxoisovalerate dehydrogenase beta subunit (Branched-Chain Keto Acid Dehydrogenase E1α), Acyl-CoA dehydrogenase, medium-chain specific, Acyl-CoA dehydrogenase, very-long-chain specific, Trifunctional enzyme alpha subunit (Long-chain 3 hydroxyacyl CoA Dehydrogenase or LCHAD) (HADHA), Trifunctional enzyme beta subunit (Hydroxyacyl-CoA Dehydrogenase/3-Ketoacyl-CoA Thiolase/Enoyl-CoA Hydratase [HADHB]), Pyruvate dehydrogenase E1 component beta subunit, and Pyruvate dehydrogenase E1 component alpha subunit.

6. The fusion protein for use of claim 1 or 2, wherein said multi-component enzyme complex is selected from the group consisting of pyruvate dehydrogenase complex (PDHC), [alpha]-ketoglutarate dehydrogenase complex (KGDHC), and branched-chain keto-acid dehydrogenase complex (BCKDHC).

7. The fusion protein for use of claim 1 or 2, wherein said multi-component enzyme complex is selected from the group consisting of complex I (NADH-ubiquinone oxidoreductase), complex II (succinateubiquinone oxidoreductase), complex III (ubiquinol-ferncytochrome c oxidoreductase), complex IV (cytochrome c oxidoreductase), and complex V (F1F0 ATPase).

8. The fusion protein for use as claimed in any one of claims 1 to 7 wherein said mitochondrial disorder is caused by a missense mutation in said enzyme.

9. The fusion protein for use as claimed in any one of claims 1 to 7 wherein said mitochondrial disorder is selected from the group consisting of LAD deficiency and isolated Complex I deficiency.

10. The fusion protein for use as claimed in any one of claims 1 to 7 wherein said mitochondrial disorder is a neurodegenerative disease.

11. The fusion protein for use as claimed in any one of claims 1 to 7 wherein said mitochondrial disorder is selected from the group consisting of encephalopathy and liver failure that is accompanied by stormy lactic acidosis, hyperammonemia and coagulopathy, Alpers Disease, Barth syndrome, Beta-oxidation Defects, Carnitine-Acyl-Carnitine Deficiency, Carnitine Deficiency, Co-Enzyme Q10 Deficiency, Complex I Deficiency, Complex II Deficiency, Complex III Deficiency, Complex IV Deficiency, Complex V Deficiency, COX Deficiency, CPEO, KSS, LCHAD, Leigh Disease or Syndrome, LHON, LIC (Lethal Infantile Cardiomyopathy), Luft Disease, MELAS, MERRF, Mitochondrial Cytopathy, Mitochondrial Myopathy, MNGIE, NARP, and Pyruvate Dehydrogenase Deficiency.

12. The fusion protein for use as claimed in any one of claims 1 to 7 wherein said mitochondrial disorder is selected from the group consisting of Ornithine Transcarbamylase deficiency (hyperammonemia) (OTCD), Carnitine Opalmitoyltransferase II deficiency (CPT2), Fumarase deficiency, Cytochrome c oxidase deficiency associated with Leigh syndrome, Maple Syrup Urine Disease (MSUD), Medium-Chain Acyl-CoA Dehydrogenase deficiency (MCAD), Acyl-CoA Dehydrogenase Very Long-Chain deficiency (LCAD), Trifunctional Protein deficiency, Progressive External Ophthalmoplegia with Mitochondrial DNA Deletions (POLG), DGUOK, TK2, Pyruvate Decarboxylase deficiency, MMC-Maternal Myopathy and Cardiomyopathy; Ataxia, Retinitis Pigmentosa; FICP-Fatal Infantile Cardiomyopathy Plus, a MELAS-associated cardiomyopathy; MELAS-Mitochondrial Encephalomyopathy with Lactic Acidosis and Strokelike episodes; LDYT-Leber's hereditary optic neuropathy and Dystonia; MHCM-Maternally inherited Hypertrophic CardioMyopathy; DM-Diabetes Mellitus; DMDF Diabetes Mellitus + Deafness; CIPO-Chronic Intestinal Pseudoobstruction with myopathy and Ophthalmoplegia; DEAF-Maternally inherited DEAFness; PEM-Progressive encephalopathy; SNHL-SensoriNeural Hearing Loss; Encephalomyopathy; DEMCHO-Dementia and Chorea; AMDF-Ataxia, Myoclonus; ESOC Epilepsy;Optic atrophy; FBSN Familial Bilateral Striatal Necrosis; FSGS Focal Segmental Glomerulosclerosis; LIMM Lethal Infantile Mitochondrial Myopathy; MDM Myopathy and Diabetes Mellitus; MEPR Myoclonic Epilepsy and Psychomotor Regression; MERME MERRF/MELAS overlap disease; MHCM Maternally Inherited Hypertrophic CardioMyopathy; MICM Maternally Inherited Cardiomyopathy; MILS Maternally Inherited Leigh Syndrome; Mitochondrial Encephalocardio myopathy; Multisystem Mitochondrial Disorder (myopathy, encephalopathy, blindness, hearing loss, peripheral neuropathy); NAION Nonarteritic Anterior Ischemic Optic Neuropathy; PEM Progressive Encephalopathy; PME Progressive Myoclonus Epilepsy; RTT Rett Syndrome; SIDS Sudden Infant Death Syndrome; and MIDD Maternally Inherited Diabetes and Deafness.

## Patentansprüche

1. Fusionsprotein zur Verwendung in einem Verfahren zur Behandlung von Mitochondriopathie in einem Individuum,
wobei das Fusionsprotein eine Proteintransduktionsdomäne (PTD) umfasst, die an eine funktionelle Komponente eines Mitochondrienenzyms und eine Mitochondrien-Targeting-Sequenz (MTS) fusioniert ist,
worin die MTS sich zwischen der PTD und der funktionellen Komponente befindet und
worin die MTS eine MTS eines anderen Mitochondrienenzyms ist, für das ein Kerngen kodiert,
worin das Mitochondrienenzym ein Enzym eines Mitochondrienmultienzymkomplexes ist,
und
worin die Proteintransduktionsdomäne ein TAT-Peptid ist,
worin die Behandlung das Verabreichen des Fusionsproteins an das Individuum umfasst und eine kontinuierliche Langzeitbehandlung für eine chronische Krankheit ist oder eine einzige oder einige Verabreichungen zur Behandlung eines akuten Leidens umfasst.

2. Fusionsprotein zur Verwendung nach Anspruch 1, worin der Teil des Fusionsproteins, der C-terminal zur MTS ist, aus der funktionellen Komponente eines Enzyms besteht.

3. Fusionsprotein zur Verwendung nach Anspruch 2, worin es keine Reste gibt, die heterolog zu dem Enzym sind, das C-terminal zur MTS vorliegt, und Spaltung der MTS ein Enzym mit der nativen Sequenz erzeugt, das leicht in einen konformationsempfindlichen Multienzymkomplex integriert wird.

4. Fusionsprotein zur Verwendung nach Anspruch 1 oder 2, worin das Enzym Lipoamiddehydrogenase (LAD) ist.

5. Fusionsprotein zur Verwendung nach Anspruch 1 oder 2, worin das Enzym aus der aus 2-Oxoisovalerat-Dehydrogenase-alpha-Untereinheit (verzweigter Ketosäuredehydrogenase E1α), 2-Oxoisovalerat-Dehydrogenase-beta-Untereinheit (verzweigter Ketosäuredehydrogenase E1α), Acyl-CoA-Dehydrogenase, mittelkettiger spezifischer Acyl-CoA-Dehydrogenase, sehr langkettiger spezifischer trifunktioneller Enzym-alpha-Untereinheit (langkettiger 3-Hydroxyacyl-CoA-Dehydrogenase oder LCHAD) (HADHA), trifunktioneller Enzym-beta-Untereinheit (Hydroxyacyl-CoA-Dehydrogenase/3-Ketoacyl-CoA-Thiolase/Enoyl-CoA-Hydratase [HADHB]), Pyruvatdehydrogenase-beta-Untereinheit der E1-Komponente und Pyruvatdehyrogenase-alphaUntereinheit der E1-Komponente bestehenden Gruppe ausgewählt ist.

6. Fusionsprotein zur Verwendung nach Anspruch 1 oder 2, worin der Multienzymkomplex aus der aus Pyruvatdehydrogenasekomplex (PDHC), [Alpha]ketoglutaratdehydrogenasekomplex (KGDHC) und verzweigtem Ketosäuredehydrogenasekomplex (BCKDHC) bestehenden Gruppe ausgewählt ist.

7. Fusionsprotein zur Verwendung nach Anspruch 1 oder 2, worin der Enzymkomplex mit mehreren Komponenten aus der aus Komplex I (NADH-Ubiquinon-Oxidoreductase), Komplex II (Succinatubiquinon-Oxidoreductase), Komplex III (Ubiquinol-Ferricytochrom-c-Oxidoreductase), Komplex IV (Cytochrom-c-Oxidoreductase) und Komplex V (F1 FO-ATPase) bestehenden Gruppe ausgewählt ist.

8. Fusionsprotein zur Verwendung nach einem der Ansprüche 1 bis 7, worin die Mitochondriopathie durch eine Fehlsinnmutation im Enzym verursacht ist.

9. Fusionsprotein zur Verwendung nach einem der Ansprüche 1 bis 7, worin die Mitochondriopathie aus der aus LAD-Defizienz und Defizienz von isoliertem Komplex I bestehenden Gruppe ausgewählt ist.

10. Fusionsprotein zur Verwendung nach einem der Ansprüche 1 bis 7, worin die Mitochondriopathie eine neurodegenerative Erkrankung ist.

11. Fusionsprotein zur Verwendung nach einem der Ansprüche 1 bis 7, worin die Mitochondriopathie aus der aus Enzephalopathie und Leberversagen, begleitet von heftiger Laktatazidose, Hyperammonämie und Koagulopathie, Alpers-Syndrom, Barth-Syndrom, Betaoxidationsdefekten, Carnitin-Acyl-Carnitindefizienz, Carnitindefizienz, Coenzym-Q10-Defizienz, Komplex-I-Defizienz, Komplex-II-Defizienz, Komplex-III-Defizienz, Komplex-IV-Defizienz, Komplex-V-Defizienz, COX-Defizienz, CPEO, KSS, LCHAD, Leigh-Krankheit oder -Syndrom, LHON, LIC (tödlicher kindlicher Kardiomyopathie), Luftscher Krankheit, MELAS, MERRF, mitochondrialer Zytopathie, mitochondrialer Myopathie, MNGIE, NARP und Pyruvatdehydrogenase-Defizienz bestehenden Gruppe ausgewählt ist.

12. Fusionsprotein zur Verwendung nach einem der Ansprüche 1 bis 7, worin die Mitochondriopathie aus der aus Ornithin-Transcarbamylase-Defizienz (Hyperammonämie) (OTCD), Carnitin-Opalmitoyltransferase-II-Defizienz (CPT2), Fumarase-Defizienz, Cytochrom-c-Oxidasedefizienz in Verbindung mit Leigh-Syndrom, Ahornsirupkrankheit (MSUD), mittelkettiger Acyl-CoA-Dehydrogenase-Defizienz (MCAD), sehr langkettiger Acyl-CoA-Dehydrogenase-Defizienz (LCAD), Defizienz von trifunktionellem Protein, progressiver externer Ophthalmoplegie mit mitochondrialen DNA-Deletionen (POLG), DGUOK, TK2, Pyruvatdecarboxylasedefizienz, MMC - maternaler Myopathie und Kardiomyopathie; Ataxie, Retinitis pigmentosa; FICP - tödlicher kindlicher Kardiomyopathie plus, einer MELAS-assoziierten Kardiomyopathie; MELAS - mitochondrialer Enzephalomyopathie mit Laktatazidose und schlaganfallähnlichen Episoden; LDYT - Leberscher hereditärer Optikusneuropathie und Dystonie; MHCM - maternal vererbter hypertropher Kardiomyopathie; DM - Diabetes mellitus; DMDF - Diabetes mellitus + Taubheit; CIPO - chronischer intestinaler Pseudoobstruktion mit Myopathie und Ophthalmoplegie; DEAF - maternal vererbter Taubheit; PEM - progressiver Enzephalopathie; SNHL - sensorineuralem Hörverlust; Enzephalomyopathie; DEMCHO - Demenz und Chorea; AMDF - Ataxie, Myoklonie; ESOC Epilepsie; optischer Atrophie; FSBN - familiärer bilateraler Stratiumnekrose; FSGS - fokal segmentaler Glomerulosklerose; LIMM - tödlicher kindlicher Mitochondrienmyopathie; MDM - Myopathie und Diabetes mellitus; MEPR - Myoklonusepilepsie und psychomotorischer Regression; MERME MERRF/MELAS Overlap-Syndrom; MHCM - maternal vererbter hypertropher Kardiomyopathie; MICM - maternal vererbter Kardiomyopathie; MILS - maternal vererbtem Leigh-Syndrom; mitochondrialer Enzephalokardiomyopathie; Multisystem-Mitochondriopathie (Myopathie, Enzephalopathie, Blindheit, Hörverlust, periphere Neuropathie); NAION nichtarteriitischer anteriorer ischämischer Optikusneuropathie, PEM progressiver Enzephalopathie; PME progressiver Myoklonusepilepsie; RTT Rett-Syndrom; SIDS plötzlichem Kindstod; und MIDD maternal vererbtem Diabetes und Taubheit bestehenden Gruppe ausgewählt ist.

## Revendications

1. Protéine de fusion pour utilisation dans un procédé de traitement d'un trouble mitochondrial chez un sujet,
ladite protéine de fusion comprenant un domaine de transduction de protéine (PTD) fusionné à un composant fonctionnel d'une enzyme mitochondriale et une séquence de ciblage mitochondrial (MTS),
ladite MTS étant située entre ledit PTD et ledit composant fonctionnel et
ladite MTS étant une MTS d'une autre enzyme mitochondriale qui est codée par un gène nucléaire,
ladite enzyme mitochondriale étant une enzyme d'un complexe enzymatique multi-composants mitochondrial, et
ledit domaine de transduction de protéine étant un peptide TAT,
ledit traitement comprenant l'administration de ladite protéine de fusion audit sujet et étant un traitement prolongé continu pour une maladie chronique ou comprend une seule administration ou quelques administrations pour le traitement d'une affection aiguë.

2. Protéine de fusion pour utilisation selon la revendication 1, **caractérisée en ce que** la partie de ladite protéine de fusion qui est C-terminale par rapport à ladite MTS est constituée dudit composant fonctionnel d'une enzyme.

3. Protéine de fusion pour utilisation selon la revendication 2 **caractérisée en ce qu'**il n'y a pas de résidus hétérologues pour l'enzyme présente en position C-terminale par rapport à la MTS et le clivage de ladite MTS génère une enzyme ayant la séquence native, qui est aisément intégrée dans un complexe enzymatique multi-composants sensible sur le plan conformationnel.

4. Protéine de fusion pour utilisation de la revendication 1 ou 2, **caractérisée en ce que** ladite enzyme est la lipoamide déshydrogénase (LAD).

5. Protéine de fusion pour utilisation de la revendication 1 ou 2, **caractérisée en ce que** ladite enzyme est choisie dans le groupe constitué des sous-unité alpha de 2-oxoisovalérate déshydrogénase (céto-acide déshydrogénase des chaînes ramifiées E1α) sous-unité bêta de 2-oxoisovalérate déshydrogénase (céto-acide déshydrogénase des chaînes ramifiées E1α), acyl-CoA déshydrogénase des acides gras à chaîne moyenne, acyl-CoA déshydrogénase des acides gras à très longue chaîne, sous-unité alpha d'enzyme trifonctionnelle (3-hydroxyacyl-CoA déshydrogénase des acides gras à longue chaîne ou LCHAD) (HADHA), sous-unité bêta d'enzyme trifonctionnelle (hydroxyacyl-CoA déshydrogénase/3- cétoacyl-CoA thiolase/énoyl-CoA hydratase [HADHB]), sous-unité bêta de composant E1 de pyruvate déshydrogénase, et sous-unité alpha de composant E1 de pyruvate déshydrogénase.

6. Protéine de fusion pour utilisation de la revendication 1 ou 2, **caractérisée en ce que** ledit complexe enzymatique multi-composants est choisi dans le groupe constitué des complexe pyruvate déshydrogénase (PDHC), complexe [alpha]-cétoglutarate déshydrogénase (KGDHC), et complexe céto-acide déshydrogénase des chaînes ramifiées (BCKDHC).

7. Protéine de fusion pour utilisation de la revendication 1 ou 2, **caractérisée en ce que** ledit complexe enzymatique multi-composants est choisi dans le groupe constitué des complexe I (NADH-ubiquinone oxydoréductase), complexe II (succinate-ubiquinone oxydoréductase), complexe III (ubiquinol-ferricytochrome C oxydoréductase), complexe IV (cytochrome C oxydoréductase), et complexe V (F1F0 ATPase).

8. Protéine de fusion pour utilisation selon l'une quelconque des revendications 1 à 7 **caractérisée en ce que** ledit trouble mitochondrial est causé par une mutation faux sens dans ladite enzyme.

9. Protéine de fusion pour utilisation selon l'une quelconque des revendications 1 à 7 **caractérisée en ce que** ledit trouble mitochondrial est choisi dans le groupe constitué d'un déficit DAL et un déficit isolé en complexe I.

10. Protéine de fusion pour utilisation selon l'une quelconque des revendications 1 à 7 **caractérisée en ce que** ledit trouble mitochondrial est une maladie neurodégénérative.

11. Protéine de fusion pour utilisation selon l'une quelconque des revendications 1 à 7 **caractérisée en ce que** ledit trouble mitochondrial est choisi dans le groupe constitué d'une encéphalopathie et une insuffisance hépatique qui est accompagnée d'une acidose lactique aiguë, une hyperammoniémie et une coagulopathie, la maladie d'Alpers, le syndrome de Barth, des défauts de bêta-oxydation, un déficit en carnitine-acyl-carnitine, un déficit en carnitine, un déficit en co-enzyme Q10, un déficit en complexe I, un déficit en complexe II, un déficit en complexe III, un déficit en complexe IV, un déficit en complexe V, un déficit en COX, CPEO, KSS, LCHAD, la maladie ou syndrome de Leigh, LHON, LIC (cardiomyopathie infantile létale), la maladie de Luft, MELAS, MERRF, une cytopathie mitochondriale, une myopathie mitochondriale, MNGIE, NARP et un déficit en pyruvate déshydrogénase.

12. Protéine de fusion pour utilisation selon l'une quelconque des revendications 1 à 7 **caractérisée en ce que** ledit trouble mitochondrial est choisi dans le groupe constitué d'un déficit en ornithine transcarbamylase (hyperammoniémie) (OTCD), un déficit en carnitine 0-palmitoyltransférase II (CPT2), un déficit en fumarase, un déficit en cytochrome C oxydase associé au syndrome de Leigh, la maladie du sirop d'érable (MSUD), un déficit en acyl-CoA déshydrogénase des acides gras à chaîne moyenne (MCAD), un déficit en acyl-CoA déshydrogénase des acides gras à très longue chaîne (LCAD), un déficit en protéine trifonctionnelle, une ophtalmoplégie externe progressive avec délétions d'ADN mitochondrial (POLG), DGUOK, TK2, un déficit en pyruvate décarboxylase, MMC - myopathie et cardiomyopathie transmises par la mère ; une ataxie, la rétinite pigmentaire ; FICP - cardiomyopathie infantile létale plus, une cardiomyopathie associée à MELAS ; une encéphalomyopathie mitochondriale associée à MELAS avec acidose lactique et épisodes de type AVC ; LDYT - neuropathie optique héréditaire de Leber et dystonie ; MHCM - cardiomyopathie hypertrophique transmise par la mère ; DM - diabète sucré ; DMDF - diabète sucré + surdité ; CIPO - pseudo-obstruction intestinale chronique avec myopathie et ophtalmoplégie ; DEAF - surdité transmise par la mère ; PEM -encéphalopathie progressive ; SNHL - perte auditive neurosensorielle ; encéphalomyopathie ; DEMCHO - démence et chorée ; AMDF - ataxie, myoclonie ; ESOC - épilepsie ; atrophie optique ; FBSN - nécrose striatale bilatérale familiale ; FSGS - glomérulosclérose segmentaire focale ; LIMM - myopathie mitochondriale infantile létale ; MDM - myopathie et diabète sucré ; MEPR - épilepsie myoclonique et régression psychomotrice ; un Overlap Syndrome MERME MERRF/MELAS ; MHCM - cardiomyopathie hypertrophique transmise par la mère ; MICM - cardiomyopathie transmise par la mère ; MILS - syndrome de Leigh transmis par la mère ; une encéphalocardiomyopathie mitochondriale ; un trouble mitochondrial multisystématisé (myopathie, encéphalopathie, cécité, perte auditive, neuropathie périphérique) ; NAION - neuropathie optique ischémique antérieure non artéritique ; PEM - encéphalopathie progressive ; PME - épilepsie myoclonique progressive ; RTT - syndrome de Rett ; SIDS - syndrome de mort subite du nourrisson ; et MIDD - diabète et surdité transmis par la mère.


### SEQUENCE LISTING

<110> Yissum Research Development Company of the Hebrew
   university of Jerusalem
   Hadasit Medical Research services and Development Ltd.
<120> METHODS AND COMPOSITIONS FOR TREATMENT OF MITOCHONDRIAL DISORDERS
<130> Yissum-001-PCT
<160> 50
<170> PatentIn version 3.5
<210> 1
   <211> 22
   <212> DNA
   <213> Artificial Sequence
<220>
   <223> artificial construct
<400> 1
   cgggatccgc agagctggag tc 22
<210> 2
   <211> 23
   <212> DNA
   <213> Artificial Sequence
<220>
   <223> artificial construct
<400> 2
   cccctcgagt caaaagttga ttg 23
<210> 3
   <211> 1629
   <212> DNA
   <213> Artificial Sequence
<220>
   <223> artificial construct
<400> 3
   atgggcagca gccatcatca tcatcatcac agcagcggcc tggtgccgcg cggcagccat 60
   atgaggaaga agcggagaca gcgacgaaga ggctcggatc cgcagagctg gagtcgtgtg 120
   tactgctcct tggccaagag aggccatttc aatcgaatat ctcatggcct acagggactt 180
   tctgcagtgc ctctgagaac ttacgcagat cagccgattg atgctgatgt aacagttata 240
   ggttctggtc ctggaggata tgttgctgct attaaagctg cccagttagg cttcaagaca 300
   gtctgcattg agaaaaatga aacacttggt ggaacatgct tgaatgttgg ttgtattcct 360
   tctaaggctt tattgaacaa ctctcattat taccatatgg cccatggaac agattttgca 420
   tctagaggaa ttgaaatgtc cgaagttcgc ttgaatttag acaagatgat ggagcagaag 480
   agtactgcag taaaagcttt aacaggtgga attgcccact tattcaaaca gaataaggtt 540
   gttcatgtca atggatatgg aaagataact ggcaaaaatc aagtcactgc tacgaaagct     600
   gatggcggca ctcaggttat tgatacaaag aacattctta tagccacggg ttcagaagtt     660
   actccttttc ctggaatcac gatagatgaa gatacaatag tgtcatctac aggtgcttta     720
   tctttaaaaa aagttccaga aaagatggtt gttattggtg caggagtaat aggtgtagaa     780
   ttgggttcag tttggcaaag acttggtgca gatgtgacag cagttgaatt tttaggtcat     840
   gtaggtggag ttggaattga tatggagata tctaaaaact ttcaacgcat ccttcaaaaa     900
   caggggttta aatttaaatt gaatacaaag gttactggtg ctaccaagaa gtcagatgga     960
   aaaattgatg tttctattga agctgcttct ggtggtaaag ctgaagttat cacttgtgat    1020
   gtactcttgg tttgcattgg ccgacgaccc tttactaaga atttgggact agaagagctg    1080
   ggaattgaac tagatcctag aggtagaatt ccagtcaata ccagatttca aactaaaatt    1140
   ccaaatatct atgccattgg tgatgtagtt gctggtccaa tgctggctca caaagcagag    1200
   gatgaaggca ttatctgtgt tgaaggaatg gctggtggtg ctgtgcacat tgactacaat    1260
   tgtgtgccat cagtgattta cacacaccct gaagttgctt gggttggcaa atcagaagag   1320
   cagttgaaag aagagggtat tgagtacaaa gttgggaaat tcccatttgc tgctaacagc    1380
   agagctaaga caaatgctga cacagatggc atggtgaaga tccttgggca gaaatcgaca    1440
   gacagagtac tgggagcaca tattcttgga ccaggtgctg gagaaatggt aaatgaagct    1500
   gctcttgctt tggaatatgg agcatcctgt gaagatatag ctagagtctg tcatgcacat    1560
   ccgaccttat cagaagcttt tagagaagca aatcttgctg cgtcatttgg caaatcaatc    1620
   aacttttga                                                            1629
<210> 4
   <211> 543
   <212> PRT
   <213> Artificial Sequence
<220>
   <223> artificial construct
<400> 4
   Met Gly Ser Ser His His His His His His Ser Ser Gly Leu Val Pro
   1               5                   10                  15
   Arg Gly Ser His Met Arg Lys Lys Arg Arg Gln Arg Arg Arg Gly Ser
               20                  25                  30
   Asp Pro Gln Ser Trp Ser Arg Val Tyr Cys Ser Leu Ala Lys Arg Gly
           35                  40                  45
   His Phe Asn Arg Ile Ser His Gly Leu Gln Gly Leu Ser Ala Val Pro
       50                  55                  60
   Leu Arg Thr Tyr Ala Asp Gln Pro Ile Asp Ala Asp Val Thr Val Ile
   65 70 75 80
   Gly Ser Gly Pro Gly Gly Tyr Val Ala Ala Ile Lys Ala Ala Gln Leu
   85 90 95
   Gly Phe Lys Thr Val Cys Ile Glu Lys Asn Glu Thr Leu Gly Gly Thr
   100 105 110
   Cys Leu Asn Val Gly Cys Ile Pro Ser Lys Ala Leu Leu Asn Asn Ser
   115 120 125
   His Tyr Tyr His Met Ala His Gly Thr Asp Phe Ala Ser Arg Gly Ile
   130 135 140
   Glu Met Ser Glu Val Arg Leu Asn Leu Asp Lys Met Met Glu Gln Lys
   145 150 155 160
   Ser Thr Ala Val Lys Ala Leu Thr Gly Gly Ile Ala His Leu Phe Lys
   165 170 175
   Gln Asn Lys Val Val His Val Asn Gly Tyr Gly Lys Ile Thr Gly Lys
   180 185 190
   Asn Gln Val Thr Ala Thr Lys Ala Asp Gly Gly Thr Gln Val Ile Asp
   195 200 205
   Thr Lys Asn Ile Leu Ile Ala Thr Gly Ser Glu Val Thr Pro Phe Pro
   210 215 220
   Gly Ile Thr Ile Asp Glu Asp Thr Ile Val Ser Ser Thr Gly Ala Leu
   225 230 235 240
   Ser Leu Lys Lys Val Pro Glu Lys Met Val Val Ile Gly Ala Gly Val
   245 250 255
   Ile Gly Val Glu Leu Gly Ser Val Trp Gln Arg Leu Gly Ala Asp Val
   260 265 270
   Thr Ala Val Glu Phe Leu Gly His Val Gly Gly Val Gly Ile Asp Met
   275 280 285
   Glu Ile Ser Lys Asn Phe Gln Arg Ile Leu Gln Lys Gln Gly Phe Lys
   290 295 300
   Phe Lys Leu Asn Thr Lys Val Thr Gly Ala Thr Lys Lys Ser Asp Gly
   305 310 315 320
   Lys Ile Asp Val Ser Ile Glu Ala Ala Ser Gly Gly Lys Ala Glu Val
                   325                 330                 335
   Ile Thr Cys Asp Val Leu Leu Val Cys Ile Gly Arg Arg Pro Phe Thr
               340                 345                 350
   Lys Asn Leu Gly Leu Glu Glu Leu Gly Ile Glu Leu Asp Pro Arg Gly
           355                 360                 365
   Arg Ile Pro Val Asn Thr Arg Phe Gln Thr Lys Ile Pro Asn Ile Tyr
       370                 375                 380
   Ala Ile Gly Asp Val Val Ala Gly Pro Met Leu Ala His Lys Ala Glu
   385                 390                 395                 400
   Asp Glu Gly Ile Ile Cys Val Glu Gly Met Ala Gly Gly Ala Val His
                   405                 410                 415
   Ile Asp Tyr Asn Cys Val Pro Ser Val Ile Tyr Thr His Pro Glu Val
               420                 425                 430
   Ala Trp Val Gly Lys Ser Glu Glu Gln Leu Lys Glu Glu Gly Ile Glu
           435                 440                 445
   Tyr Lys Val Gly Lys Phe Pro Phe Ala Ala Asn Ser Arg Ala Lys Thr
       450                 455                 460
   Asn Ala Asp Thr Asp Gly Met Val Lys Ile Leu Gly Gln Lys Ser Thr
   465                 470                 475                 480
   Asp Arg Val Leu Gly Ala His Ile Leu Gly Pro Gly Ala Gly Glu Met
                   485                 490                 495
   Val Asn Glu Ala Ala Leu Ala Leu Glu Tyr Gly Ala Ser Cys Glu Asp
               500                 505                 510
   Ile Ala Arg Val Cys His Ala His Pro Thr Leu Ser Glu Ala Phe Arg
           515                 520                 525
   Glu Ala Asn Leu Ala Ala Ser Phe Gly Lys Ser Ile Asn Phe Glx
       530                 535                 540
<210> 5
   <211> 27
   <212> DNA
   <213> Artificial Sequence
<220>
   <223> artificial construct
<400> 5
   cgggatccgg cagatcagcc gattgat 27
<210> 6
   <211> 23
   <212> DNA
   <213> Artificial Sequence
<220>
   <223> artificial construct
<400> 6
   cccctcgagt caaaagttga ttg 23
<210> 7
   <211> 1458
   <212> DNA
   <213> Homo sapiens
<400> 7
   acttgtgcgc atgctccggg tgtcccggag ttgtcctgcg ccggtgttcc cacgtgcggc      60
   ctgaacctga gcgcataatg ttatgaggag atgggagcac tagtgattcg cggtatcagg     120
   aatttcaacc tagagaaccg agcggaacgg gaaatcagca agatgaagcc ctctgtcgct     180
   cccagacacc cctctaccaa cagcctcctg cgagagcaga ttagtctcta tccagaagtt     240
   aaaggagaga ttgctcgtaa agatgaaaag ctgctgtcgt ttctaaaaga tgtgtatgtt     300
   gattccaaag atcctgtgtc ttccttgcag gtaaaagctg ctgaaacatg tcaagagccg     360
   aaggaattca gattgccgaa agaccatcat tttgatatga taaatattaa gagcattccc     420
   aaaggcaaaa tttccattgt agaagcattg acacttctca ataatcataa gcttttccca     480
   gaaacctgga ctgctgagaa aataatgcag gaataccagt tagaacagaa agatgtgaat     540
   tctcttctta aatattttgt tacttttgaa gtcgaaatct tccctcctga agacaagaaa     600
   gcaatacgat caaaatgaag aaaatcacag aaatttccta tgtgtactcc tcatccctcc     660
   tgctgtatat tttctcattt tttgcgtaat aaattatgtt aattaccaaa tatttaatgc     720
   tctcattgtg agggcatact cttaatattt attgagctcc ctgacttttc aagattgcca     780
   tagaatatct tttgttttat tttaatttgg tttaggcata tttcataagt gcatgtcagc     840
   atgactgagc acatctgtac ctttattatg gtgaaagagt taatttgtta ttttaggcac     900
   atataccaac ttttaaattg gtcatattac cccttgggaa atgtcctgaa tccctcaact     960
   gagttttact ttccaagtta ggtgtttagt tcattcttct catgcgatag tgaaagtaaa    1020
   agctttcctg acccttaaga ctgacatttt ctcgttaggg aaagagactc gatagcaggg    1080
   tagactaaca gtagttccca aataaaggca cacaggagaa aatgaatcaa tagaatctaa    1140
   ttaaaattac tactccaggg aataagaagt attgaggatt tattttattt tgaattggtt    1200
   ttaagtatgg aataattgtc tttacagtta taaaaagtaa ttagtggtgt ttacaaaatg    1260
   ttgggaattt ctgctttgac agataggtaa gtcccatggg aatttctgct ttgacaaata    1320
   gttaagtccc atggccttca gagaggcagg aagcagaaac tttcaaaaaa gtaacatttc    1380
   tctggattta atgtggaaat agagaaaaat ttaaagaata attaaaaaac catggtagat    1440
   gaaatttggt gattacat                                                  1458
<210> 8
   <211> 175
   <212> PRT
   <213> Homo sapiens
<400> 8
   Met Gly Ala Leu Val Ile Arg Gly Ile Arg Asn Phe Asn Leu Glu Asn
   1               5                   10                  15
   Arg Ala Glu Arg Glu Ile Ser Lys Met Lys Pro Ser Val Ala Pro Arg
               20                  25                  30
   His Pro Ser Thr Asn Ser Leu Leu Arg Glu Gln Ile Ser Leu Tyr Pro
           35                  40                  45
   Glu Val Lys Gly Glu Ile Ala Arg Lys Asp Glu Lys Leu Leu Ser Phe
       50                  55                  60
   Leu Lys Asp Val Tyr Val Asp Ser Lys Asp Pro Val Ser Ser Leu Gln
   65                  70                  75                  80
   Val Lys Ala Ala Glu Thr Cys Gln Glu Pro Lys Glu Phe Arg Leu Pro
                   85                  90                  95
   Lys Asp His His Phe Asp Met Ile Asn Ile Lys Ser Ile Pro Lys Gly
               100                 105                 110
   Lys Ile Ser Ile Val Glu Ala Leu Thr Leu Leu Asn Asn His Lys Leu
           115                 120                 125
   Phe Pro Glu Thr Trp Thr Ala Glu Lys Ile Met Gln Glu Tyr Gln Leu
       130                 135                 140
   Glu Gln Lys Asp Val Asn Ser Leu Leu Lys Tyr Phe Val Thr Phe Glu
   145                 150                 155                 160
   Val Glu Ile Phe Pro Pro Glu Asp Lys Lys Ala Ile Arg Ser Lys
                   165                 170                 175
<210> 9
   <211> 34
   <212> PRT
   <213> Homo sapiens
<400> 9
   Met Gly Ala Leu Val Ile Arg Gly Ile Arg Asn Phe Asn Leu Glu Asn
   1               5                   10                  15
   Arg Ala Glu Arg Glu Ile Ser Lys Met Lys Pro Ser Val Ala Pro Arg
               20                  25                  30
   His Pro
<210> 10
   <211> 13
   <212> PRT
   <213> Human immunodeficiency virus type 1
<400> 10
   Gly Arg Lys Lys Arg Arg Gln Arg Arg Arg Pro Pro Gln
   1 5 10
<210> 11
   <211> 34
   <212> PRT
   <213> herpes simplex virus 7
<400> 11
   Asp Ala Ala Thr Ala Thr Arg Gly Arg Ser Ala Ala Ser Arg Pro Thr
   1 5 10 15
   Glu Arg Pro Arg Ala Pro Ala Arg Ser Ala Ser Arg Pro Arg Arg Pro
   20 25 30
   Val Asp
<210> 12
   <211> 16
   <212> PRT
   <213> Latimeria menadoensis
<400> 12
   Arg Gln Ile Lys Ile Trp Phe Gln Asn Arg Arg Met Lys Trp Lys Lys
   1 5 10 15
<210> 13
   <211> 27
   <212> PRT
   <213> Artificial Sequence
<220>
   <223> synthetic construct
<400> 13
   Gly Trp Thr Leu Asn Ser Ala Gly Tyr Leu Leu Gly Lys Ile Asn Leu
   1 5 10 15
   Lys Ala Leu Ala Ala Leu Ala Lys Lys Ile Leu
   20 25
<210> 14
   <211> 18
   <212> PRT
   <213> Unknown
<220>
   <223> unknown source
<400> 14
   Lys Leu Ala Leu Lys Leu Ala Leu Lys Ala Leu Lys Ala Ala Leu Lys
   1               5                   10                  15
   Leu Ala
<210> 15
   <211> 30
   <212> PRT
   <213> unknown
<220>
   <223> unknown source
<400> 15
   Trp Glu Ala Lys Leu Ala Lys Ala Leu Ala Lys Ala Leu Ala Lys His
   1               5                   10                  15
   Leu Ala Lys Ala Leu Ala Lys Ala Leu Lys Ala Cys Glu Ala
               20                  25                  30
<210> 16
   <211> 509
   <212> PRT
   <213> Homo sapiens
<400> 16
<210> 17
   <211> 20
   <212> PRT
   <213> unknown
<220>
   <223> unknown source
<400> 17
   Gly Leu Phe Arg Ala Leu Leu Arg Leu Leu Arg Ser Leu Trp Arg Leu
   1               5                   10                  15
   Leu Leu Arg Ala
               20
<210> 18
   <211> 18
   <212> PRT
   <213> Artificial Sequence
<220>
   <223> synthetic construct
<400> 18
   Val Arg Leu Pro Pro Pro Val Arg Leu Pro Pro Pro Val Arg Leu Pro
   1               5                   10                  15
   Pro Pro
<210> 19
   <211> 27
   <212> PRT
   <213> Caiman crocodilus
<400> 19
   Met Gly Leu Gly Leu His Leu Leu Val Leu Ala Ala Ala Leu Gln Gly
   1               5                   10                  15
   Ala Trp Ser Gln Pro Lys Lys Lys Arg Lys Val
               20                  25
<210> 20
   <211> 27
   <212> PRT
   <213> Unknown
<220>
   <223> unknown source
<400> 20
   Gly Ala Leu Phe Leu Gly Phe Leu Gly Ala Ala Gly Ser Thr Met Gly
   1               5                   10                  15
   Ala Trp Ser Gln Pro Lys Lys Lys Arg Lys Val
               20                  25
<210> 21
   <211> 21
   <212> PRT
   <213> unknown
<220>
   <223> unknown source
<400> 21
   Lys Glu Thr Trp Trp Glu Thr Trp Trp Thr Glu Trp Ser Gln Pro Lys
   1               5                   10                  15
   Lys Lys Arg Lys Val
               20
<210> 22
   <211> 17
   <212> PRT
   <213> Human immunodeficiency virus type 1
<400> 22
   Thr Arg Gln Ala Arg Arg Asn Arg Arg Arg Arg Trp Arg Glu Arg Gln
   1               5                   10                  15
   Arg
<210> 23
   <211> 15
   <212> PRT
   <213> Feline herpesvirus 1
<400> 23
   Arg Arg Arg Arg Asn Arg Thr Arg Arg Asn Arg Arg Arg Val Arg
   1               5                   10                  15
<210> 24
   <211> 19
   <212> PRT
   <213> Brome mosaic virus
<400> 24
   Lys Met Thr Arg Ala Gln Arg Arg Ala Ala Ala Arg Arg Asn Arg Trp
   1               5                   10                  15
   Thr Ala Arg
<210> 25
   <211> 13
   <212> PRT
   <213> Human lymphotropic virus type III
<400> 25
   Thr Arg Arg Gln Arg Thr Arg Arg Ala Arg Arg Asn Arg
   1               5                   10
<210> 26
   <211> 22
   <212> PRT
   <213> Cabassous unicinctus
<400> 26
   Lys Leu Thr Arg Ala Gln Arg Arg Ala Ala Ala Arg Lys Asn Lys Arg
   1               5                   10                  15
   Asn Lys Arg Asn Thr Arg
               20
<210> 27
   <211> 17
   <212> PRT
   <213> Enterobacteria phage P22
<400> 27
   Asn Ala Lys Thr Arg Arg His Glu Arg Arg Arg Lys Leu Ala Ile Glu
   1               5                   10                  15
   Arg
<210> 28
   <211> 18
   <212> PRT
   <213> unknown
<220>
   <223> unknown source
<400> 28
   Thr Ala Lys Thr Arg Tyr Lys Ala Arg Arg Ala Glu Leu Ile Ala Glu
   1               5                   10                  15
   Arg Arg
<210> 29
   <211> 16
   <212> PRT
   <213> saccharomyces cerevisiae
<400> 29
   Thr Arg Arg Asn Lys Arg Asn Arg Ile Gln Glu Gln Leu Asn Arg Lys
   1               5                   10                  15
<210> 30
   <211> 26
   <212> PRT
   <213> Homo sapiens
<400> 30
   Lys Arg Arg Ile Arg Arg Glu Arg Asn Lys Met Ala Ala Ala Lys Ser
   1               5                   10                  15
   Arg Asn Arg Arg Arg Glu Leu Thr Asp Arg
               20                  25
<210> 31
   <211> 28
   <212> PRT
   <213> Homo sapiens
<400> 31
   Arg Ile Lys Ala Glu Arg Lys Arg Met Arg Asn Arg Ile Ala Ala Ser
   1               5                   10                  15
   Lys Ser Arg Lys Arg Lys Leu Glu Arg Ile Ala Arg
               20                  25
<210> 32
   <211> 22
   <212> PRT
   <213> Saccharomyces cerevisiae
<400> 32
   Lys Arg Ala Arg Asn Thr Glu Ala Ala Arg Arg Ser Arg Ala Arg Lys
   1               5                   10                  15
   Leu Gln Arg Met Lys Gln
               20
<210> 33
   <211> 9
   <212> PRT
   <213> Artificial Sequence
<220>
   <223> synthetic construct
<400> 33
   Arg Arg Arg Arg Arg Arg Arg Arg Arg
   1               5
<210> 34
   <211> 24
   <212> PRT
   <213> Artificial sequence
<220>
   <223> synthetic construct
<400> 34
   Leu Gly Thr Tyr Thr Gln Asp Phe Asn Lys Phe His Thr Phe Pro Gln
   1 5 10 15
   Thr Ala Ile Gly Val Gly Ala Pro
   20
<210> 35
   <211> 11
   <212> PRT
   <213> Acidithiobacillus ferrooxidans
<400> 35
   Tyr Ala Arg Ala Ala Ala Arg Gln Ala Arg Ala
   1 5 10
<210> 36
   <211> 21
   <212> PRT
   <213> Artificial Sequence
<220>
   <223> synthetic construct
<400> 36
   Lys Glu Thr Trp Trp Glu Thr Trp Trp Thr Glu Trp Ser Gln Pro Lys
   1 5 10 15
   Lys Lys Arg Lys Val
   20
<210> 37
   <211> 16
   <212> PRT
   <213> Drosophila melanogaster
<400> 37
   Arg Gln Ile Lys Ile Trp Phe Gln Asn Arg Arg Met Lys Trp Lys Lys
   1 5 10 15
<210> 38
   <211> 11
   <212> PRT
   <213> Artificial Sequence
<220>
   <223> synthetic construct
<400> 38
   Arg Arg Arg Arg Arg Arg Arg Arg Arg Arg Arg
   1 5 10
<210> 39
   <211> 35
   <212> PRT
   <213> Homo sapiens
<400> 39
   Met Gln Ser Trp Ser Arg Val Tyr Cys Ser Leu Ala Lys Arg Gly His
   1               5                   10                  15
   Phe Asn Arg Ile Ser His Gly Leu Gln Gly Leu Ser Ala Val Pro Leu
               20                  25                  30
   Arg Thr Tyr
           35
<210> 40
   <211> 25
   <212> PRT
   <213> Homo sapiens
<400> 40
   Met Leu Ser Ala Leu Ala Arg Pro Val Ser Ala Ala Leu Arg Arg Ser
   1               5                   10                  15
   Phe Ser Thr Ser Ala Gln Asn Asn Ala
               20                  25
<210> 41
   <211> 34
   <212> PRT
   <213> Homo sapiens
<400> 41
   Gln Ser Trp Ser Arg Val Tyr Cys Ser Leu Ala Lys Arg Gly His Phe
   1               5                   10                  15
   Asn Arg Ile Ser His Gly Leu Gln Gly Leu Ser Ala Val Pro Leu Arg
               20                  25                  30
   Thr Tyr
<210> 42
   <211> 13
   <212> PRT
   <213> Artificial Sequence
<220>
   <223> synthetic construct
<400> 42
   Gly Trp Thr Leu Asn Ser Ala Gly Tyr Leu Leu Gly Pro
   1 5 10
<210> 43
   <211> 16
   <212> PRT
   <213> Homo sapiens
<400> 43
   Ala Ala Val Ala Leu Leu Pro Ala Val Leu Leu Ala Leu Leu Ala Pro
   1 5 10 15
<210> 44
   <211> 18
   <212> PRT
   <213> Homo sapiens
<400> 44
   Leu Leu Ile Ile Leu Arg Arg Arg Ile Arg Lys Gln Ala His Ala His
   1 5 10 15
   Ser Lys
<210> 45
   <211> 1599
   <212> DNA
   <213> Homo sapiens
<400> 45
   atgggcagca gccatcatca tcatcatcac agcagcggcc tggtgccgcg cggcagccat 60
   atgtcggatc cgcagagctg gagtcgtgtg tactgctcct tggccaagag aggccatttc 120
   aatcgaatat ctcatggcct acagggactt tctgcagtgc ctctgagaac ttacgcagat 180
   cagccgattg atgctgatgt aacagttata ggttctggtc ctggaggata tgttgctgct 240
   attaaagctg cccagttagg cttcaagaca gtctgcattg agaaaaatga aacacttggt 300
   ggaacatgct tgaatgttgg ttgtattcct tctaaggctt tattgaacaa ctctcattat 360
   taccatatgg cccatggaac agattttgca tctagaggaa ttgaaatgtc cgaagttcgc 420
   ttgaatttag acaagatgat ggagcagaag agtactgcag taaaagcttt aacaggtgga 480
   attgcccact tattcaaaca gaataaggtt gttcatgtca atggatatgg aaagataact 540
   ggcaaaaatc aagtcactgc tacgaaagct gatggcggca ctcaggttat tgatacaaag 600
   aacattctta tagccacggg ttcagaagtt actccttttc ctggaatcac gatagatgaa 660
   gatacaatag tgtcatctac aggtgcttta tctttaaaaa aagttccaga aaagatggtt 720
   gttattggtg caggagtaat aggtgtagaa ttgggttcag tttggcaaag acttggtgca 780
   gatgtgacag cagttgaatt tttaggtcat gtaggtggag ttggaattga tatggagata 840
   tctaaaaact ttcaacgcat ccttcaaaaa caggggttta aatttaaatt gaatacaaag 900
   gttactggtg ctaccaagaa gtcagatgga aaaattgatg tttctattga agctgcttct 960
   ggtggtaaag ctgaagttat cacttgtgat gtactcttgg tttgcattgg ccgacgaccc 1020
   tttactaaga atttgggact agaagagctg ggaattgaac tagatcctag aggtagaatt 1080
   ccagtcaata ccagatttca aactaaaatt ccaaatatct atgccattgg tgatgtagtt 1140
   gctggtccaa tgctggctca caaagcagag gatgaaggca ttatctgtgt tgaaggaatg 1200
   gctggtggtg ctgtgcacat tgactacaat tgtgtgccat cagtgattta cacacaccct 1260
   gaagttgctt gggttggcaa atcagaagag cagttgaaag aagagggtat tgagtacaaa 1320
   gttgggaaat tcccatttgc tgctaacagc agagctaaga caaatgctga cacagatggc 1380
   atggtgaaga tccttgggca gaaatcgaca gacagagtac tgggagcaca tattcttgga 1440
   ccaggtgctg gagaaatggt aaatgaagct gctcttgctt tggaatatgg agcatcctgt 1500
   gaagatatag ctagagtctg tcatgcacat ccgaccttat cagaagcttt tagagaagca 1560
   aatcttgctg cgtcatttgg caaatcaatc aacttttga 1599
<210> 46
   <211> 532
   <212> PRT
   <213> Homo sapiens
<400> 46
   Met Gly Ser Ser His His His His His His Ser Ser Gly Leu Val Pro
   1               5                   10                  15
   Arg Gly Ser His Met Ser Asp Pro Gln Ser Trp Ser Arg Val Tyr Cys
               20                  25                  30
   Ser Leu Ala Lys Arg Gly His Phe Asn Arg Ile Ser His Gly Leu Gln
           35                  40                  45
   Gly Leu Ser Ala Val Pro Leu Arg Thr Tyr Ala Asp Gln Pro Ile Asp
       50                  55                  60
   Ala Asp Val Thr Val Ile Gly Ser Gly Pro Gly Gly Tyr Val Ala Ala
   65                  70                  75                      80
   Ile Lys Ala Ala Gln Leu Gly Phe Lys Thr Val Cys Ile Glu Lys Asn
                   85                  90                  95
   Glu Thr Leu Gly Gly Thr Cys Leu Asn Val Gly Cys Ile Pro Ser Lys
               100                 105                 110
   Ala Leu Leu Asn Asn Ser His Tyr Tyr His Met Ala His Gly Thr Asp
           115                 120                 125
   Phe Ala Ser Arg Gly Ile Glu Met Ser Glu Val Arg Leu Asn Leu Asp
   130 135 140
   Lys Met Met Glu Gln Lys Ser Thr Ala Val Lys Ala Leu Thr Gly Gly
   145 150 155 160
   Ile Ala His Leu Phe Lys Gln Asn Lys Val Val His Val Asn Gly Tyr
   165 170 175
   Gly Lys Ile Thr Gly Lys Asn Gln Val Thr Ala Thr Lys Ala Asp Gly
   180 185 190
   Gly Thr Gln Val Ile Asp Thr Lys Asn Ile Leu Ile Ala Thr Gly Ser
   195 200 205
   Glu Val Thr Pro Phe Pro Gly Ile Thr Ile Asp Glu Asp Thr Ile Val
   210 215 220
   Ser Ser Thr Gly Ala Leu Ser Leu Lys Lys Val Pro Glu Lys Met Val
   225 230 235 240
   Val Ile Gly Ala Gly Val Ile Gly Val Glu Leu Gly Ser Val Trp Gln
   245 250 255
   Arg Leu Gly Ala Asp Val Thr Ala Val Glu Phe Leu Gly His Val Gly
   260 265 270
   Gly Val Gly Ile Asp Met Glu Ile Ser Lys Asn Phe Gln Arg Ile Leu
   275 280 285
   Gln Lys Gln Gly Phe Lys Phe Lys Leu Asn Thr Lys Val Thr Gly Ala
   290 295 300
   Thr Lys Lys Ser Asp Gly Lys Ile Asp Val Ser Ile Glu Ala Ala Ser
   305 310 315 320
   Gly Gly Lys Ala Glu Val Ile Thr Cys Asp Val Leu Leu Val Cys Ile
   325 330 335
   Gly Arg Arg Pro Phe Thr Lys Asn Leu Gly Leu Glu Glu Leu Gly Ile
   340 345 350
   Glu Leu Asp Pro Arg Gly Arg Ile Pro Val Asn Thr Arg Phe Gln Thr
   355 360 365
   Lys Ile Pro Asn Ile Tyr Ala Ile Gly Asp Val Val Ala Gly Pro Met
   370 375 380
   Leu Ala His Lys Ala Glu Asp Glu Gly Ile Ile Cys Val Glu Gly Met
   385 390 395 400
   Ala Gly Gly Ala Val His Ile Asp Tyr Asn Cys Val Pro Ser Val Ile
   405 410 415
   Tyr Thr His Pro Glu Val Ala Trp Val Gly Lys Ser Glu Glu Gln Leu
   420 425 430
   Lys Glu Glu Gly Ile Glu Tyr Lys Val Gly Lys Phe Pro Phe Ala Ala
   435 440 445
   Asn Ser Arg Ala Lys Thr Asn Ala Asp Thr Asp Gly Met Val Lys Ile
   450 455 460
   Leu Gly Gln Lys Ser Thr Asp Arg Val Leu Gly Ala His Ile Leu Gly
   465 470 475 480
   Pro Gly Ala Gly Glu Met Val Asn Glu Ala Ala Leu Ala Leu Glu Tyr
   485 490 495
   Gly Ala Ser Cys Glu Asp Ile Ala Arg Val Cys His Ala His Pro Thr
   500 505 510
   Leu Ser Glu Ala Phe Arg Glu Ala Asn Leu Ala Ala Ser Phe Gly Lys
   515 520 525
   Ser Ile Asn Phe
   530
<210> 47
   <211> 24
   <212> DNA
   <213> Artificial Sequence
<220>
   <223> synthetic construct
<400> 47
   cgggatccgg gagcactagt gatt 24
<210> 48
   <211> 27
   <212> DNA
   <213> Artificial sequence
<220>
   <223> synthetic construct
<400> 48
   cccctcgagt cattttgatc gtattgc 27
<210> 49
   <211> 31
   <212> PRT
   <213> Homo sapiens
<400> 49
   Met Pro Ala Arg Ala Leu Leu Pro Arg Arg Met Gly His Arg Thr Leu
   1 5 10 15
   Ala Ser Thr Pro Ala Leu Trp Ala Ser Ile Pro Cys Pro Arg Ser
   20 25 30
<210> 50
   <211> 55
   <212> PRT
   <213> Homo sapiens
<400> 50
   Met Tyr Arg Tyr Leu Ala Lys Ala Leu Leu Pro Ser Arg Ala Gly Pro
   1 5 10 15
   Ala Ala Leu Gly Ser Ala Ala Asn His Ser Ala Ala Leu Leu Gly Arg
   20 25 30
   Gly Arg Gly Gln Pro Ala Ala Ala Ser Gln Pro Gly Leu Ala Leu Ala
   35 40 45
   Ala Arg Arg His Tyr Ser Glu
   50 55